(19) Europäisches Patentamt
European Patent Office
Office européen des brevets

(11) **EP 4 502 599 A1**

(12) **EUROPEAN PATENT APPLICATION**
published in accordance with Art. 153(4) EPC

(43) Date of publication:
05.02.2025 Bulletin 2025/06

(21) Application number: 22933598.9

(22) Date of filing: 17.10.2022

(51) International Patent Classification (IPC):
*G01N 33/48* (2006.01)    *C12M 1/00* (2006.01)
*C12N 1/02* (2006.01)    *C12N 5/09* (2010.01)

(52) Cooperative Patent Classification (CPC):
C12M 1/00; C12N 1/02; C12N 5/06; G01N 33/48

(86) International application number:
PCT/JP2022/038554

(87) International publication number:
WO 2023/181468 (28.09.2023 Gazette 2023/39)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 25.03.2022 JP 2022049652

(71) Applicant: Sekisui Medical Co., Ltd.
Tokyo 103-0027 (JP)

(72) Inventors:
• UCHIYAMA, Takaya
  Shunan-shi, Yamaguchi 746-0006 (JP)
• KANDA, Marika
  Shunan-shi, Yamaguchi 746-0006 (JP)
• KOMAI, Kuniya
  Shunan-shi, Yamaguchi 746-0006 (JP)
• INOUE, Tomonori
  Shunan-shi, Yamaguchi 746-0006 (JP)

(74) Representative: Ter Meer Steinmeister & Partner
Patentanwälte mbB
Nymphenburger Straße 4
80335 München (DE)

(54) **KIT FOR ISOLATING CIRCULATING TUMOR CELLS, CONTAINER FOR ISOLATING CIRCULATING TUMOR CELLS, AND METHOD FOR ISOLATING CIRCULATING TUMOR CELLS**

(57) Provided is a circulating tumor cell isolation kit capable of increasing a recovery rate of circulating tumor cells not only when a specimen for which a long time has not elapsed since blood collection is used but also when a specimen stored for several days after blood collection is used. The circulating tumor cell isolation kit according to the present invention is a kit used for isolation of circulating tumor cells in blood, the kit including: a blood collection container accommodating an aqueous solution and collecting a predetermined amount of blood; and a cell isolation container accommodating a cell isolation material having a specific gravity at 25°C of 1.065 to 1.080, the aqueous solution containing an anticoagulant and containing a low molecular weight compound having a molecular weight of 75 to 500 or a high molecular weight compound having a number average molecular weight of 2,000 or more and less than 200,000, and when physiological saline in an amount equivalent to the predetermined amount of blood collected in the blood collection container is collected in the blood collection container to obtain a mixed liquid in which the physiological saline and the aqueous solution are mixed, an osmotic pressure of the mixed liquid being 270 to 350 mOsm/L.

**EP 4 502 599 A1**

[FIG. 1]

**Description**

**TECHNICAL FIELD**

The present invention relates to a circulating tumor cell isolation kit used for isolation of circulating tumor cells in blood, and a method for isolating circulating tumor cells using the same. The present invention also relates to a circulating tumor cell isolation container used for isolation of circulating tumor cells in blood, and a method for isolating circulating tumor cells using the same.

**BACKGROUND ART**

Circulating tumor cells (hereinafter, they may be referred to as "CTCs") are cancer cells that have been released from tumor tissue and infiltrated into blood, and are thought to be deeply involved in a process of forming cancer metastasis. For example, it has been reported that CTCs can serve as prognostic predictors and markers of a degree of progression in lung cancer, and stratification markers of a risk of recurrence of breast cancer.

However, it is not easy to isolate CTCs in blood since only a few to several tens of CTCs are present per 1 mL of blood of a patient.

In order to isolate CTCs in blood, various methods have been attempted as shown in Non-Patent Document 1 below.

In addition, Patent Document 1 below discloses a composition containing a component capable of releasing an aldehyde, an anticoagulant, and cyclodextrin or a functionalized derivative thereof. Patent Document 1 describes that CTCs stabilized with the above composition may be isolated. For example, Patent Document 1 describes that CTCs and white blood cells may be isolated from buffy coats after isolation of plasma.

**Related Art Documents**

**Patent Document**

Patent Document 1: JP 2021-500585 T

**Non-Patent Document**

Non-Patent Document 1: Zeina Habli, Walid AlChamaa, Raya Saab, Humam Kadara and Massoud L. Khraiche. Circulating Tumor Cell Detection Technologies and Clinical Utility: Challenges and Opportunities. Cancers 2020, 12, 1930; doi: 10.3390/cancers12071930

**SUMMARY OF THE INVENTION**

**PROBLEMS TO BE SOLVED BY THE INVENTION**

As an example of a method for isolating CTCs in blood, as described in Non-Patent Document 1 and Patent Document 1, a centrifugation method using a difference in specific gravity of cells (density gradient centrifugation method) is known. The centrifugation method has advantages of low cost and being easy to use.

Meanwhile, in a clinical setting, a certain period of time may elapse before a specimen collected from a patient is subjected to testing. For example, when a specimen is transported to a laboratory or a large amount of specimens waiting for testing is present in the laboratory, a time of about several days may elapse from when blood is collected until the specimen is subjected to testing.

In a conventional centrifugation method, when CTCs are isolated using a specimen for which a long time has not elapsed since blood collection, a recovery rate of CTCs can be increased to some extent, but when CTCs are isolated using a specimen stored for several days after blood collection, the recovery rate of CTCs is likely to decrease.

An object of the present invention is to provide a circulating tumor cell isolation kit and a circulating tumor cell isolation container capable of increasing a recovery rate of circulating tumor cells not only when a specimen for which a long time has not elapsed since blood collection is used but also when a specimen stored for several days after blood collection is used. Another object of the present invention is to provide a method for isolating circulating tumor cells using the above circulating tumor cell isolation kit and a method for isolating circulating tumor cells using the above circulating tumor cell isolation container.

**MEANS FOR SOLVING THE PROBLEMS**

In a broad aspect of the present invention, there is provided a circulating tumor cell isolation kit used for isolation of circulating tumor cells in blood, the circulating tumor cell isolation kit including: a blood collection container accommodating an aqueous solution and collecting a predetermined amount of blood; and a cell isolation container accommodating a cell isolation material having a specific gravity at 25°C of 1.065 or more and 1.080 or less, the aqueous solution containing an anticoagulant and containing a low molecular weight compound having a molecular weight of 75 or more and 500 or less or a high molecular weight compound having a number average molecular weight of 2,000 or more and less than 200,000, and when physiological saline in an amount equivalent to the predetermined amount of blood collected in the blood collection container is collected in the blood collection container to obtain a mixed liquid in which the physiological saline and the aqueous solution are mixed, an osmotic pressure of the mixed liquid being 270 mOsm/L or more and 350 mOsm/L or less (hereinafter, it may be referred to as a "CTC isolation kit").

In a specific aspect of the CTC isolation kit according to the present invention, the aqueous solution contains the low molecular weight compound.

In a specific aspect of the CTC isolation kit according to the present invention, the aqueous solution contains the high molecular weight compound.

In a specific aspect of the CTC isolation kit according to the present invention, the aqueous solution contains the low molecular weight compound and the high molecular weight compound.

In a specific aspect of the CTC isolation kit according to the present invention, the low molecular weight compound contains trehalose or ammonium sulfate.

In a specific aspect of the CTC isolation kit according to the present invention, the high molecular weight compound contains a compound having a cell membrane protective action.

In a specific aspect of the CTC isolation kit according to the present invention, the high molecular weight compound is dextran, polyethylene glycol, or polyvinylpyrrolidone.

In a specific aspect of the CTC isolation kit according to the present invention, the cell isolation material is a composition for cell isolation.

In a specific aspect of the CTC isolation kit according to the present invention, the composition for cell isolation contains an organic component having fluidity at 25°C and an inorganic fine powder, the organic component contains a resin, and the inorganic fine powder contains fine powder silica.

In a specific aspect of the CTC isolation kit according to the present invention, the CTC isolation kit further includes a hemagglutinating agent.

In a broad aspect of the present invention, there is provided a method for isolating circulating tumor cells in blood using the above-described circulating tumor cell isolation kit, the method including: a blood collection step of collecting blood in the blood collection container accommodating the aqueous solution to obtain a specimen in which the blood and the aqueous solution are mixed; an addition step of adding the specimen to the cell isolation container accommodating the cell isolation material; a centrifugation step of centrifuging the cell isolation container to which the specimen has been added; and a recovery step of recovering circulating tumor cells isolated in plasma (hereinafter, it may be referred to as a "CTC isolation method").

In a specific aspect of the CTC isolation method according to the present invention, in the centrifugation step, a cell isolation container accommodating a liquid in which a hemagglutinating agent and the specimen are mixed is centrifuged.

In a broad aspect of the present invention, there is provided a circulating tumor cell isolation container used for isolation of circulating tumor cells in blood, the circulating tumor cell isolation container being a circulating tumor cell isolation container in which a predetermined amount of blood is collected, the circulating tumor cell isolation container including: a container main body; an aqueous solution accommodated in the container main body; and a cell isolation material accommodated in the container main body and having a specific gravity at 25°C of 1.065 or more and 1.080 or less, the aqueous solution containing an anticoagulant and containing a low molecular weight compound having a molecular weight of 75 or more and 500 or less or a high molecular weight compound having a number average molecular weight of 2,000 or more and less than 200,000, and when physiological saline in an amount equivalent to the predetermined amount of blood collected in the circulating tumor cell isolation container is collected in the circulating tumor cell isolation container to obtain a mixed liquid in which the physiological saline and the aqueous solution are mixed, an osmotic pressure of the mixed liquid being 270 mOsm/L or more and 350 mOsm/L or less (hereinafter, it may be referred to as a "CTC isolation container").

In a specific aspect of the CTC isolation container according to the present invention, the aqueous solution contains the low molecular weight compound.

In a specific aspect of the CTC isolation container according to the present invention, the aqueous solution contains the high molecular weight compound.

In a specific aspect of the CTC isolation container according to the present invention, the aqueous solution contains the low molecular weight compound and the high molecular weight compound.

In a specific aspect of the CTC isolation container according to the present invention, the low molecular weight compound contains trehalose or ammonium sulfate.

In a specific aspect of the CTC isolation container according to the present invention, the high molecular weight compound contains a compound having a cell membrane protective action.

In a specific aspect of the CTC isolation container according to the present invention, the high molecular weight compound is dextran, polyethylene glycol, or polyvinylpyrrolidone.

In a specific aspect of the CTC isolation container according to the present invention, the cell isolation material is a composition for cell isolation.

In a specific aspect of the CTC isolation container according to the present invention, the composition for cell isolation contains an organic component having fluidity at 25°C and an inorganic fine powder, the organic component contains a resin, and the inorganic fine powder contains fine powder silica.

In a broad aspect of the present invention, there is provided a circulating tumor cell isolation kit, including: the above-described circulating tumor cell isolation container; and a hemagglutinating agent.

In a broad aspect of the present invention, there is provided a method for isolating circulating tumor cells in blood using the above-described circulating tumor cell isolation container, the method including: a blood collection step of collecting blood in the circulating tumor cell isolation container to obtain a specimen in which the blood and the aqueous solution are mixed; a centrifugation step of centrifuging the circulating tumor cell isolation container accommodating the specimen; and a recovery step of recovering circulating tumor cells isolated in plasma (hereinafter, it may be referred to as a "CTC isolation method").

In a specific aspect of the CTC isolation method according to the present invention, in the centrifugation step, a circulating tumor cell isolation container accommodating a liquid in which a hemagglutinating agent and the specimen are mixed is centrifuged.

## EFFECT OF THE INVENTION

According to the present invention, it is possible to provide a circulating tumor cell isolation kit and a circulating tumor cell isolation container capable of increasing a recovery rate of circulating tumor cells not only when a specimen for which a long time has not elapsed since blood collection is used but also when a specimen stored for several days after blood collection is used.

## BRIEF DESCRIPTION OF DRAWINGS

[Fig. 1] Fig. 1 is a front cross-sectional view schematically showing a circulating tumor cell isolation kit according to an embodiment of the present invention.

[Fig. 2] Fig. 2 is a front cross-sectional view schematically showing a circulating tumor cell isolation container according to an embodiment of the present invention.

## MODES FOR CARRYING OUT THE INVENTION

Hereinafter, the present invention will be described in detail.

A circulating tumor cell isolation kit (hereinafter, it may be referred to as a "CTC isolation kit") according to the present invention is used for isolation of circulating tumor cells in blood. The CTC isolation kit according to the present invention includes a blood collection container accommodating an aqueous solution and collecting a predetermined amount of blood and a cell isolation container accommodating a cell isolation material having a specific gravity at 25°C of 1.065 or more and 1.080 or less. In the CTC isolation kit according to the present invention, the above aqueous solution contains an anticoagulant and contains a low molecular weight compound having a molecular weight of 75 or more and 500 or less or a high molecular weight compound having a number average molecular weight of 2,000 or more and less than 200,000. In the CTC isolation kit according to the present invention, when physiological saline in an amount equivalent to the predetermined amount of blood collected in the above blood collection container is collected in the blood collection container to obtain a mixed liquid in which the above physiological saline and the above aqueous solution are mixed, an osmotic pressure of the above mixed liquid is 270 mOsm/L or more and 350 mOsm/L or less.

A circulating tumor cell isolation container (hereinafter, it may be referred to as a "CTC isolation container") according to the present invention is used for isolation of circulating tumor cells in blood. The CTC isolation container according to the present invention is a circulating tumor cell isolation container in which a predetermined amount of blood is collected. The CTC isolation container according to the present invention includes a container main body, an aqueous solution accommodated in the above container main body, and a cell isolation material accommodated in the above container main body and having a specific gravity at 25°C of 1.065 or more and 1.080 or less. In the CTC isolation container according to the present invention, the above aqueous solution contains an anticoagulant and contains a low molecular weight compound having a molecular weight of 75 or more and 500 or less or a high molecular weight compound having a number average molecular weight of 2,000 or more and less than 200,000. In the CTC isolation container according to the present invention, when physiological saline in an amount equivalent to the predetermined amount of blood collected in the above CTC isolation container is collected in the CTC isolation container to obtain a mixed liquid in which the above physiological saline and the above aqueous solution are mixed, an osmotic pressure of the above mixed liquid is 270 mOsm/L or more and 350 mOsm/L or less.

Since the CTC isolation kit and the CTC isolation container according to the present invention are provided with the above configurations, it is possible to increase a recovery rate of circulating tumor cells (CTCs) not only when a specimen for which a long time has not elapsed since blood collection is used but also when a specimen stored for several days after blood collection is used.

In the CTC isolation kit according to the present invention, when blood is collected in the blood collection container, a mixed liquid (specimen) in which the above blood and the above aqueous solution are mixed is obtained. Since the osmotic pressure of the mixed liquid is moderately small, CTCs are easily stabilized. In addition, CTCs are easily stabilized by an action of the above low molecular weight compound or the above high molecular weight compound contained in the above aqueous solution. Therefore, in the CTC isolation kit according to the present invention, it is possible to increase the recovery rate of CTCs not only when a specimen for which a long time has not elapsed since blood collection is used but also when a specimen stored for several days after blood collection is used. Also in the CTC isolation container according to the present invention, it is possible to increase the recovery rate of CTCs not only when a specimen for which a long time has not elapsed since blood collection is used but also when a specimen stored for several days after blood collection is used, by a similar action.

In addition, in a conventional method of isolating CTCs in blood by centrifugation, when a specimen is stored between blood collection and centrifugation operation, cell death and change in cell morphology proceed during storage. Therefore, as the storage time becomes longer, the number of red blood cells and the number of white blood cells mixed on a partition formed by an isolation material increase after centrifugation. In observation with a fluorescence microscope or analysis with NGS, it is preferable that the purity of cancer cells is high, and thus, when the number of mixed blood cells increases, detection sensitivity of CTCs decreases. Furthermore, as in Patent Document 1 above, when CTCs are isolated from buffy coats after centrifugation, unnecessary cells such as white blood cells are recovered together with CTCs, and thus, it is necessary to remove unnecessary cells using a cell sorter or the like, and operation becomes complicated.

On the other hand, since the CTC isolation kit and the CTC isolation container according to the present invention are provided with the above configurations, it is possible to suppress cell death and change in cell morphology during storage, and it is also possible to suppress mixing of white blood cells. Therefore, in the CTC isolation kit and the CTC isolation container according to the present invention, CTCs can be easily and stably isolated at a high recovery rate.

Specific embodiments of the present invention will be described with reference to the drawings.

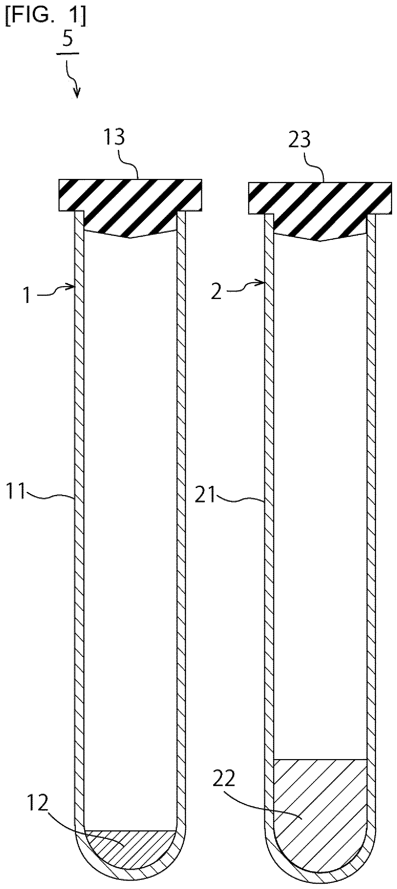

Fig. 1 is a front cross-sectional view schematically showing a circulating tumor cell isolation kit according to an embodiment of the present invention.

The CTC isolation kit 5 shown in Fig. 1 includes a blood collection container 1 and a cell isolation container 2.

The blood collection container 1 includes a blood collection container main body 11, an aqueous solution 12, and a plug 13. The blood collection container main body 11 has an opening at one end and a bottom closed at the other end. The aqueous solution 12 is accommodated in the blood collection container main body 11. The aqueous solution 12 contains an anticoagulant and contains a low molecular weight compound having a molecular weight of 75 or more and 500 or less or a high molecular weight compound having a number average molecular weight of 2,000 or more and less than 200,000. The plug 13 is inserted into the opening of the blood collection container main body 11.

The cell isolation container 2 includes a cell isolation container main body 21, a cell isolation material 22, and a plug 23. The cell isolation container main body 21 has an opening at one end and a bottom closed at the other end. The cell isolation material 22 is accommodated in the cell isolation container main body 21. The specific gravity at 25°C of the cell isolation material 22 is 1.065 or more and 1.080 or less. The plug 23 is inserted into the opening of the cell isolation container main body 21.

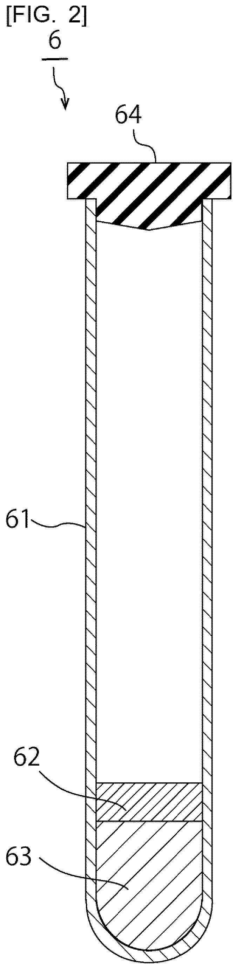

Fig. 2 is a front cross-sectional view schematically showing a circulating tumor cell isolation container according to an embodiment of the present invention.

The CTC isolation container 6 shown in Fig. 2 includes a container main body 61, an aqueous solution 62, a cell isolation material 63, and a plug 64. The container main body 61 has an opening at one end and a bottom closed at the other end. The aqueous solution 62 and the cell isolation material 63 are accommodated in the container main body 61. The aqueous solution 62 contains an anticoagulant and contains a low molecular weight compound having a molecular weight of 75 or more and 500 or less or a high molecular weight compound having a number average molecular weight of 2,000 or more and less than 200,000. The specific gravity at 25°C of the cell isolation material 63 is 1.065 or more and 1.080 or less.

The plug 64 is inserted into the opening of the container main body 61.

Next, the above aqueous solution and the above cell isolation material used in the present invention will be described.

(Aqueous solution)

The above aqueous solution contains an anticoagulant and contains a low molecular weight compound having a molecular weight of 75 or more and 500 or less (hereinafter, it may be referred to as "low molecular weight compound (A)") or a high molecular weight compound having a number average molecular weight of 2,000 or more and less than 200,000 (hereinafter, it may be referred to as "high molecular weight compound (B)"). Therefore, the above aqueous solution contains an anticoagulant and contains low molecular weight compound (A) or high molecular weight compound (B). The above aqueous solution preferably contains an anticoagulant and water. The solute contained in the above aqueous solution contains an anticoagulant. The solute contained in the above aqueous solution contains low molecular weight compound (A) or high molecular weight compound (B). From the viewpoint of more effectively exhibiting the effect of the present invention, the above aqueous solution preferably contains low molecular weight compound (A) and high molecular weight compound (B).

<Anticoagulant>

The above aqueous solution contains an anticoagulant. As the above anticoagulant, a conventionally known anticoagulant can be used. Only one kind of the above anticoagulant may be used, or two or more kinds thereof may be used in combination.

Examples of the above anticoagulant include heparin, a metal salt of heparin, ethylenediaminetetraacetic acid (EDTA), a metal salt of EDTA, and citric acid.

From the viewpoint of favorably exhibiting anticoagulation performance, the above anticoagulant is preferably EDTA, a metal salt of EDTA, heparin, a metal salt of heparin, or sodium citrate.

The concentration of the above anticoagulant in the above aqueous solution is preferably 2 mM or more, more preferably 5 mM or more, still more preferably 10 mM or more, preferably 2000 mM or less, more preferably 1000 mM or less, still more preferably 500 mM or less, yet more preferably 250 mM or less, further more preferably 100 mM or less, particularly preferably 50 mM or less. When the concentration of the above anticoagulant is the above lower limit or more and the above upper limit or less, anticoagulation performance can be favorably exhibited.

<Low molecular weight compound having molecular weight of 75 or more and 500 or less>

The above aqueous solution preferably contains a low molecular weight compound having a molecular weight of 75 or more and 500 or less (low molecular weight compound (A)}. Low molecular weight compound (A) is a compound different from the anticoagulant. By using low molecular weight compound (A), the osmotic pressure is easily adjusted. Only one kind of low molecular weight compound (A) may be used, or two or more kinds thereof may be used in combination.

The molecular weight of low molecular weight compound (A) may be 90 or more, 200 or more, 350 or less, or 250 or less.

The molecular weight of low molecular weight compound (A) means, when a structural formula of the low molecular weight compound (A) can be specified, a molecular weight that can be calculated from the structural formula, and indicates, when a structural formula of the low molecular weight compound (A) cannot be specified, a number average molecular weight in terms of polystyrene measured by gel permeation chromatography (GPC).

Low molecular weight compound (A) preferably contains a compound having a cell membrane protective action. In this case, since the stability of CTCs can be further enhanced, the recovery rate of CTCs can be further increased even when a specimen stored for several days after blood collection is used.

Examples of low molecular weight compound (A) include inorganic salts, saccharides, sugar alcohols, ammonium sulfate, adenine, and propylene glycol.

Examples of the above inorganic salt include sodium salts such as sodium hydrogen phosphate and potassium salts such as potassium hydrogen carbonate.

Examples of the above saccharide include glucose, trehalose, dihydroxyacetone, fructose, galactose, sucrose, maltose, and lactulose. Trehalose is a compound having a cell membrane protective action.

Examples of the above sugar alcohol include inositol, D-mannitol, and D-sorbitol.

Low molecular weight compound (A) preferably contains trehalose, ammonium sulfate, glucose, adenine, or inositol, more preferably contains trehalose or ammonium sulfate, and still more preferably contains trehalose and ammonium sulfate. Low molecular weight compound (A) preferably contains trehalose, and also preferably contains ammonium sulfate. In this case, the effect of the present invention can be more effectively exhibited.

The content of low molecular weight compound (A) in 100 wt% of the above aqueous solution is preferably 0.01 wt% or more, more preferably 0.1 wt% or more, still more preferably 1 wt% or more, preferably 10 wt% or less, more preferably 9.5 wt% or less, still more preferably 7 wt% or less, particularly preferably 5 wt% or less. When the content of low molecular weight compound (A) is the above lower limit or more and the above upper limit or less, the effect of the present invention can be more effectively exhibited.

When the above aqueous solution contains trehalose, the content of trehalose in 100 wt% of the above aqueous solution is preferably 0.5 wt% or more, more preferably 1 wt% or more, still more preferably 2 wt% or more, preferably 9 wt% or less, more preferably 8 wt% or less, still more preferably 6 wt% or less. When the above content of trehalose is the above lower limit or more and the above upper limit or less, CTCs can be further stabilized, and the effect of the present invention can be more effectively exhibited.

When the above aqueous solution contains ammonium sulfate, the content of ammonium sulfate in 100 wt% of the above aqueous solution is preferably 0.01 wt% or more, more preferably 0.1 wt% or more, still more preferably 1 wt% or more, preferably 9 wt% or less, more preferably 8 wt% or less, still more preferably 6 wt% or less. When the above content of ammonium sulfate is the above lower limit or more and the above upper limit or less, CTCs can be further stabilized, and the effect of the present invention can be more effectively exhibited.

When the above aqueous solution contains glucose, the content of glucose in 100 wt% of the above aqueous solution is preferably 0.1 wt% or more, more preferably 0.3 wt% or more, preferably 10 wt% or less, more preferably 5 wt% or less. When the above content of glucose is the above lower limit or more and the above upper limit or less, the effect of the present invention can be more effectively exhibited. In particular, when the above content of glucose is the above lower limit or more, a nutrient source is supplied to CTCs, so that the survival rate of CTCs can be increased, and as a result, the effect of the present invention can be more effectively exhibited.

When the above aqueous solution contains adenine, the content of adenine in 100 wt% of the above aqueous solution is preferably 0.01 wt% or more, more preferably 0.05 wt% or more, preferably 9 wt% or less, more preferably 5 wt% or less. When the above content of adenine is the above lower limit or more and the above upper limit or less, the effect of the present invention can be more effectively exhibited. In particular, when the above content of adenine is the above lower limit or more, a nutrient source is supplied to CTCs, so that the survival rate of CTCs can be increased, and as a result, the effect of the present invention can be more effectively exhibited.

When the above aqueous solution contains inositol, the content of inositol in 100 wt% of the above aqueous solution is preferably 0.01 wt% or more, more preferably 0.05 wt% or more, preferably 9 wt% or less, more preferably 5 wt% or less. When the above content of inositol is the above lower limit or more and the above upper limit or less, the effect of the present invention can be more effectively exhibited. In particular, when the above content of inositol is the above lower limit or more, a nutrient source is supplied to CTCs, so that the survival rate of CTCs can be increased, and as a result, the effect of the present invention can be more effectively exhibited.

The total content of low molecular weight compound (A) and water in 100 wt% of the above aqueous solution is preferably 75 wt% or more, more preferably 80 wt% or more, still more preferably 85 wt% or more, preferably 99.5 wt% or less. When the above total content is the above lower limit or more and the above upper limit or less, the effect of the present invention can be more effectively exhibited.

<High molecular weight compound having number average molecular weight of 2,000 or more and less than 200,000>

The above aqueous solution preferably contains a high molecular weight compound having a number average molecular weight of 2,000 or more and less than 200,000 (2,000 or more and less than 200,000) (high molecular weight compound (B)). High molecular weight compound (B) is a compound different from the anticoagulant. The reason why the effect of the present invention is exhibited by using high molecular weight compound (B) is presumed to be that high molecular weight compound (B) effectively protects the cell membrane of CTCs to stabilize CTCs, but is not limited thereto. Only one kind of high molecular weight compound (B) may be used, or two or more kinds thereof may be used in combination.

The number average molecular weight of high molecular weight compound (B) is preferably 3,000 or more, more preferably 10,000 or more, still more preferably 20,000 or more, preferably 150,000 or less, more preferably 100,000 or less. When the number average molecular weight of high molecular weight compound (B) is the above lower limit or more and the above upper limit or less, CTCs can be further stabilized, and the recovery rate of CTCs can be further increased even when a specimen stored for several days after blood collection is used.

The number average molecular weight of high molecular weight compound (B) indicates a number average molecular weight in terms of polystyrene measured by gel permeation chromatography (GPC).

High molecular weight compound (B) is preferably a compound having no hemagglutination action. In this case, the effect of the present invention can be more effectively exhibited.

High molecular weight compound (B) preferably contains a compound having a cell membrane protective action. In this case, since the stability of CTCs can be further enhanced, the recovery rate of CTCs can be further increased even when a specimen stored for several days after blood collection is used.

High molecular weight compound (B) preferably contains a high molecular weight compound having a number average molecular weight of 10,000 or more and less than 200,000 (hereinafter, it may be referred to as high molecular weight compound (B1)). High molecular weight compound (B) more preferably contains high molecular weight compound (B1) and a high molecular weight compound having a number average molecular weight of 2,000 or more and less than 10,000 (hereinafter, it may be referred to as high molecular weight compound (B2)). In this case, the effect of the present invention can be more effectively exhibited.

The number average molecular weight of high molecular weight compound (B1) is 10,000 or more and less than 200,000, preferably 20,000 or more, more preferably 30,000 or more, preferably 150,000 or less, more preferably 100,000 or less. In this case, the effect of the present invention can be more effectively exhibited.

The number average molecular weight of high molecular weight compound (B2) is 2,000 or more and less than 10,000, preferably 3,000 or more, preferably 8,000 or less, more preferably 5,000 or less. In this case, the effect of the present invention can be more effectively exhibited.

Examples of high molecular weight compound (B) include dextran, polyethylene glycol, polyvinylpyrrolidone, polyvinyl alcohol, methyl cellulose, hydroxyethyl cellulose, and corn starch.

High molecular weight compound (B) is preferably dextran, polyethylene glycol, or polyvinylpyrrolidone. High molecular weight compound (B1) is preferably dextran, polyethylene glycol, or polyvinylpyrrolidone. High molecular weight compound (B2) is preferably dextran, polyethylene glycol, or polyvinylpyrrolidone. In this case, CTCs can be further stabilized, and the recovery rate of CTCs can be further increased even when a specimen stored for several days after blood collection is used.

Each content of high molecular weight compound (B) in 100 wt% of the above aqueous solution is preferably 0.01 wt% or more, more preferably 0.1 wt% or more, still more preferably 1 wt% or more, preferably 15 wt% or less, more preferably 10 wt% or less, still more preferably 8 wt% or less. When each content of high molecular weight compound (B) is the above lower limit or more and the above upper limit or less, CTCs can be further stabilized, and the recovery rate of CTCs can be further increased even when a specimen stored for several days after blood collection is used. The above phrase "each content of high molecular weight compound (B)" means, when the above aqueous solution contains one kind of high molecular weight compound (B), the content of the high molecular weight compound (B), and when the above aqueous solution contains two or more kinds of high molecular weight compounds (B), the content of each of the high molecular weight compounds (B).

The content of high molecular weight compound (B) in 100 wt% of the above aqueous solution is preferably 0.01 wt% or more, more preferably 0.1 wt% or more, still more preferably 1 wt% or more, preferably 20 wt% or less, more preferably 15 wt% or less, still more preferably 10 wt% or less. When the content of high molecular weight compound (B) is the above lower limit or more and the above upper limit or less, CTCs can be further stabilized, and the recovery rate of CTCs can be further increased even when a specimen stored for several days after blood collection is used.

The total content of high molecular weight compound (B) and water in 100 wt% of the above aqueous solution is preferably 75 wt% or more, more preferably 80 wt% or more, still more preferably 85 wt% or more, particularly preferably 90 wt% or more, preferably 99.5 wt% or less. When the above total content is the above lower limit or more and the above upper limit or less, the effect of the present invention can be more effectively exhibited.

The total content of low molecular weight compound (A), high molecular weight compound (B), and water in 100 wt% of the above aqueous solution is preferably 80 wt% or more, more preferably 90 wt% or more, still more preferably 95 wt% or more, particularly preferably 98 wt% or more, preferably 99.5 wt% or less. When the above total content is the above lower limit or more and the above upper limit or less, the effect of the present invention can be more effectively exhibited.

<Other components>

The above aqueous solution may contain components other than the above-described components (anticoagulant, low molecular weight compound (A), and high molecular weight compound (B)) as long as the effect of the present invention is not impaired.

(Cell isolation material)

As the above cell isolation material, a conventionally known cell isolation material can be used. The cell isolation material has a specific gravity capable of forming a partition between plasma containing CTCs and blood cells such as red blood cells after centrifugation. Examples of the above cell isolation material include a composition for cell isolation and a jig for cell isolation. The above cell isolation material is preferably the above composition for cell isolation because it is easy to produce the cell isolation material.

From the viewpoint of forming a partition between CTCs and blood cells after centrifugation and favorably isolating the CTCs and the blood cells, the specific gravity at 25°C of the above cell isolation material is 1.065 or more and 1.080 or less.

The specific gravity at 25°C of the above cell isolation material is preferably 1.067 or more, more preferably 1.070 or more, preferably 1.079 or less, more preferably 1.078 or less, still more preferably 1.077 or less, particularly preferably 1.075 or less, most preferably 1.073 or less. When the specific gravity at 25°C of the above cell isolation material is the above lower limit or more and the above upper limit or less, CTCs and blood cells can be more favorably isolated, and contamination of plasma by blood cells can be more effectively suppressed.

<Composition for cell isolation>

The above composition for cell isolation is a composition that moves between a plasma layer and a blood cell layer during centrifugation to form a partition. The above composition for cell isolation preferably has thixotropy.

The above composition for cell isolation preferably contains an organic component having fluidity at 25°C and an inorganic fine powder. Only one kind of each of the above organic component having fluidity at 25°C and the above inorganic fine powder may be used, or two or more kinds thereof may be used in combination.

Organic component having fluidity at 25°C:
The above phrase "having fluidity at 25°C" means that the viscosity at 25°C is 500 Pa·s or less.

The viscosity at 25°C of the above organic component is preferably 10 Pa·s or more, more preferably 30 Pa·s or more, preferably 350 Pa·s or less, more preferably 200 Pa·s or less. When the above viscosity is the above lower limit or more and the above upper limit or less, the fluidity of the composition for cell isolation is enhanced, and the strength of the partition formed by centrifugation operation can be enhanced.

The viscosity at 25°C of the above organic component is measured using an E-type viscometer (for example, "TVE-35" manufactured by Toki Sangyo Co., Ltd.) under the conditions of 25°C and a shear rate of 1.0 second$^{-1}$.

Examples of the above organic component include a resin and a mixture of a resin and an organic compound such as a plasticizer. Therefore, the above organic component preferably contains the above resin, and more preferably contains the above resin and the above organic compound. When the above organic component is a mixture of the above resin and the above organic compound, the resin or the organic compound may not have fluidity as long as the mixture (the above organic component) has fluidity. When the above organic component is a mixture of the above resin and the above organic compound, the resin may be, for example, a resin that is solid at 25°C. Only one kind of each of the above resin and the above organic compound may be used, or two or more kinds thereof may be used in combination.

Examples of the above resin include a petroleum resin, a cyclopentadiene resin, a polyester resin, a polyurethane resin, a (meth)acrylic resin, a silicone resin, an α-olefin-fumaric acid ester copolymer, a copolymer of sebacic acid, 2,2-dimethyl-1,3-propanediol, and 1,2-propanediol, a polyether polyurethane resin, and a polyether polyester resin. Only one kind of the above resin may be used, or two or more kinds thereof may be used in combination.

The above resin preferably contains a petroleum resin, a cyclopentadiene resin, a polyester resin, or a (meth) acrylic resin.

Examples of commercially available products of the above petroleum resin include "Regalite S5090" manufactured by Eastman Chemical Company.

Examples of the above cyclopentadiene resin include a polymer of a cyclopentadiene monomer, a copolymer of a cyclopentadiene monomer and an aromatic monomer, and a dicyclopentadiene resin. The above cyclopentadiene resin may be hydrogenated. The above polymer of a cyclopentadiene monomer and the above copolymer of a cyclopentadiene monomer and an aromatic monomer may be oligomers.

Examples of the above cyclopentadiene monomer include cyclopentadiene, dicyclopentadiene, and an alkyl-substituted derivative of cyclopentadiene.

Examples of the above aromatic monomer include styrene, methylstyrene, indene, and methylindene.

Examples of commercially available products of the above dicyclopentadiene resin include "SUKOREZ SU500" and "SUKOREZ SU90" manufactured by Kolon Industries, Inc.

Examples of the above polyester resin include a polyalkylene terephthalate resin and a polyalkylene naphthalate resin. Examples of the above polyalkylene terephthalate resin include polyethylene terephthalate, polybutylene terephthalate, and poly-1,4-cyclohexanedimethylene terephthalate.

Examples of the above polyurethane resin include a reaction product of a polyol compound and an isocyanate compound.

Examples of the above (meth)acrylic resin include a resin obtained by polymerizing at least one (meth)acrylic acid ester monomer, and a resin obtained by polymerizing at least one (meth)acrylic acid ester monomer and at least one monomer other than the (meth)acrylic acid ester monomer.

Examples of the above (meth)acrylic acid ester monomer include a (meth)acrylic acid alkyl ester, a (meth)acrylic acid polyalkylene glycol ester, a (meth)acrylic acid alkoxyalkyl ester, a (meth)acrylic acid hydroxyalkyl ester, a (meth)acrylic acid glycidyl ester, a (meth)acrylic acid dialkylaminoalkyl ester, a (meth)acrylic acid benzyl ester, a (meth)acrylic acid phenoxyalkyl ester, a (meth)acrylic acid cyclohexyl ester, a (meth)acrylic acid isobornyl ester, and a (meth)acrylic acid alkoxysilylalkyl ester. When the above (meth)acrylic acid ester monomer has an alkyl group, the number of carbon atoms of the alkyl group is preferably 1 or more and preferably 20 or less. The above (meth)acrylic acid alkyl ester is preferably a (meth)acrylic acid alkyl ester having an alkyl group having 1 to 20 carbon atoms. Only one kind of the above (meth)acrylic acid ester monomer may be used, or two or more kinds thereof may be used in combination.

Examples of the above organic compound include a benzene polycarboxylic acid alkyl ester derivative. The above organic compound is preferably a benzene polycarboxylic acid alkyl ester derivative. Therefore, the above organic component is preferably a mixture of the above resin and the above benzene polycarboxylic acid alkyl ester derivative.

Examples of the above benzene polycarboxylic acid alkyl ester derivative include a phthalic acid ester, a trimellitic acid ester, and a pyromellitic acid ester. Only one kind of the above benzene polycarboxylic acid alkyl ester derivative may be used, or two or more kinds thereof may be used in combination.

Examples of the above trimellitic acid ester include tri-n-octyl trimellitate, triisooctyl trimellitate, and triisodecyl trimellitate.

Examples of the above pyromellitic acid ester include tetraisooctyl pyromellitate.

Examples of commercially available products of the above trimellitic acid ester include "MONOCIZER W700" and "MONOCIZER W-750" manufactured by DIC Corporation, and "SANSO CIZER TOTM" and "SANSO CIZER TITM" manufactured by New Japan Chemical Co., Ltd.

Examples of commercially available products of the above pyromellitic acid ester include "MONOCIZER W-7010" manufactured by DIC Corporation.

The above benzene polycarboxylic acid alkyl ester derivative is preferably a phthalic acid ester, a trimellitic acid ester, or a pyromellitic acid ester, more preferably a trimellitic acid ester.

The content of the above organic component in 100 wt% of the above composition for cell isolation is preferably 80 wt% or more, more preferably 85 wt% or more, still more preferably 90 wt% or more, preferably 97 wt% or less.

Inorganic fine powder:

Examples of the above inorganic fine powder include fine powder silica, titanium oxide powder, calcium carbonate powder, zinc oxide powder, alumina powder, glass fine powder, talc powder, kaolin powder, bentonite powder, titania powder, and zirconium powder.

From the viewpoint of maintaining both the specific gravity and the thixotropy of the composition for cell isolation in a suitable range, the above inorganic fine powder preferably contains fine powder silica. From the viewpoint of effectively increasing the specific gravity of the composition for cell isolation, the above inorganic fine powder more preferably contains fine powder silica and an inorganic fine powder other than the fine powder silica (second inorganic fine powder). Only one kind of each of the above inorganic fine powder, the above fine powder silica, and the above second inorganic fine powder may be used, or two or more kinds thereof may be used in combination.

Examples of the above fine powder silica include natural silica and synthetic silica. Examples of the synthetic silica include hydrophilic silica and hydrophobic silica. The hydrophilic silica has an action of imparting thixotropy to the composition for cell isolation and adjusting the specific gravity, for example, by hydrogen bonding between hydroxyl groups on the particle surface. On the other hand, the hydrophobic silica has a smaller effect of imparting thixotropy than that of the hydrophilic silica.

From the viewpoint of maintaining both the specific gravity and the thixotropy of the composition for cell isolation in a suitable range, the above fine powder silica preferably contains hydrophilic silica, more preferably contains hydrophilic silica and hydrophobic silica.

The above second inorganic fine powder is preferably an inorganic fine powder having a specific gravity larger than that of fine powder silica, more preferably an inorganic fine powder having a specific gravity of 2.5 or more, such as a zinc oxide powder, a titanium oxide powder, or an alumina powder.

The specific gravity of the above second inorganic fine powder is preferably 2.5 or more, more preferably 3 or more, still more preferably 3.5 or more, particularly preferably 4 or more. The specific gravity of the above second inorganic fine powder is preferably as large as possible. When the above specific gravity is the above lower limit or more, the specific gravity of the composition for cell isolation can be effectively increased.

The average particle diameter of the above inorganic fine powder, the above fine powder silica, and the above second inorganic fine powder is not particularly limited. The average particle diameter of the above inorganic fine powder, the above fine powder silica, and the above second inorganic fine powder may be 1 nm or more, 10 nm or more, 500 nm or less, or 100 nm or less.

The average particle diameter of the above inorganic fine powder, the above fine powder silica, and the above second inorganic fine powder is an average diameter measured on a volume basis, and is a value of a median diameter of 500 (D50). The above volume average particle diameter (D50) can be measured by a laser diffraction/scattering method, an image analysis method, a Coulter method, a centrifugal sedimentation method, or the like. The above volume average particle diameter (D50) is preferably determined by measurement by a laser diffraction/scattering method or an image analysis method.

The specific surface area of the above fine powder silica is not particularly limited. The specific surface area of the above fine powder silica may be 20 $m^2$/g or more, 100 $m^2$/g or more, 500 $m^2$/g or less, or 300 $m^2$/g or less.

The specific surface area of the above fine powder silica is measured by a BET method.

The content of the above hydrophilic silica in 100 wt% of the above composition for cell isolation is preferably 0.01 wt% or more, more preferably 0.10 wt% or more, still more preferably 0.30 wt% or more, preferably 2.50 wt% or less, more preferably 2.00 wt% or less. When the content of the above hydrophilic silica is the above lower limit or more and the above upper limit or less, both the specific gravity and the thixotropy of the composition for cell isolation can be maintained in a more suitable range.

The content of the above fine powder silica in 100 wt% of the above composition for cell isolation is preferably 0.1 wt% or more, more preferably 0.5 wt% or more, preferably 10 wt% or less, more preferably 7 wt% or less. When the content of the above fine powder silica is the above lower limit or more and the above upper limit or less, both the specific gravity and the thixotropy of the composition for cell isolation can be maintained in a more suitable range.

The content of the above second inorganic fine powder in 100 wt% of the above composition for cell isolation is preferably 0.01 wt% or more, more preferably 0.1 wt% or more, preferably 10 wt% or less, more preferably 7 wt% or less. When the content of the above second inorganic fine powder is the above lower limit or more and the above upper limit or less, the specific gravity of the composition for cell isolation can be effectively increased.

The content of the above inorganic fine powder in 100 wt% of the above composition for cell isolation is preferably 0.1 wt% or more, more preferably 0.5 wt% or more, preferably 10 wt% or less, more preferably 7 wt% or less. When the content of the above inorganic fine powder is the above lower limit or more and the above upper limit or less, the specific gravity of the composition for cell isolation can be effectively increased.

Other components:

The above composition for cell isolation may contain components other than the above-described components as long as the effect of the present invention is not impaired. Examples of the above other components include an organic gelling agent, a thermoplastic elastomer, a polyalkylene glycol, a silicone oil, an auxiliary solvent, an antioxidant, a colorant, and water. Only one kind of each of the above other components may be used, or two or more kinds thereof may be used in combination.

The specific gravity at 25°C of the above composition for cell isolation is 1.065 or more and 1.080 or less. The specific gravity at 25°C of the above composition for cell isolation is preferably 1.067 or more, more preferably 1.070 or more, preferably 1.079 or less, more preferably 1.078 or less, still more preferably 1.077 or less, particularly preferably 1.075 or less, most preferably 1.073 or less. When the specific gravity at 25°C of the above composition for cell isolation is the above lower limit or more and the above upper limit or less, CTCs and blood cells such as red blood cells can be more favorably isolated, and contamination of plasma by blood cells can be more effectively suppressed.

The specific gravity at 25°C of the above composition for cell isolation is measured by sequentially adding one drop of the composition for cell isolation into saline at 25°C whose specific gravity is adjusted stepwise at intervals of 0.002, and by flotation and sedimentation in the saline.

The viscosity at 25°C of the above composition for cell isolation is preferably 50 Pa.s or more, more preferably 70 Pa.s or more, preferably 500 Pa·s or less, more preferably 400 Pa·s or less. When the above viscosity is the above lower limit or more and the above upper limit or less, the effect of the present invention can be more effectively exhibited.

The viscosity at 25°C of the above composition for cell isolation is measured using an E-type viscometer (for example, "TVE-35" manufactured by Toki Sangyo Co., Ltd.) under the conditions of 25°C and a shear rate of 1.0 second$^{-1}$.

<Jig for cell isolation>

The above jig for cell isolation is a jig that moves between a plasma layer and a blood cell layer during centrifugation to form a partition.

Examples of the above jig for cell isolation include a mechanical separator (jig for cell isolation) described in WO 2010/132783 A1 and the like.

Examples of the material of the above jig for cell isolation include elastomer.

(Circulating tumor cell (CTC) isolation kit)

The above CTC isolation kit includes a blood collection container and a cell isolation container. The above CTC isolation kit preferably further includes a hemagglutinating agent as described later.

<Blood collection container>

The above blood collection container includes a blood collection container main body and the above aqueous solution accommodated in the above blood collection container main body. Therefore, the above aqueous solution is accommodated in the above blood collection container. In addition, a predetermined amount of blood is collected in the above blood collection container.

The amount of the aqueous solution accommodated in the above blood collection container main body is appropriately changed according to the size of the blood collection container main body, the amount of blood collected, and the like. The amount of the aqueous solution accommodated in the above blood collection container main body is preferably 0.1 mL or more, more preferably 0.5 mL or more, still more preferably 0.7 mL or more, preferably 5 mL or less, more preferably 3 mL or less, still more preferably 2.5 mL or less. When the above amount of the aqueous solution is the above lower limit or more and the above upper limit or less, the effect of the present invention can be more effectively exhibited without excessively diluting the blood.

The above blood collection container is a blood collection container in which a predetermined amount of blood is collected. The above predetermined amount of the above blood is appropriately changed depending on the size, internal pressure, and the like of the blood collection container. The above predetermined amount of the above blood may be 1 mL or more, 2 mL or more, 4 mL or more, 12 mL or less, 11 mL or less, or 10 mL or less.

Physiological saline in an amount equivalent to the predetermined amount of the blood collected in the above blood collection container is collected in the above blood collection container to obtain a mixed liquid in which the above physiological saline and the above aqueous solution are mixed. For example, in the blood collection container in which 5 mL of blood is collected, 5 mL of physiological saline is collected in the blood collection container and mixed by inversion or the like to mix the above physiological saline and the above aqueous solution to obtain a mixed liquid. From the viewpoint of exhibiting the effect of the present invention, in the above CTC isolation kit, the osmotic pressure of the above mixed liquid in which the above physiological saline and the above aqueous solution are mixed is 270 mOsm/L or more and 350 mOsm/L or less. The fact that the osmotic pressure of the above mixed liquid is 270 mOsm/L or more and 350 mOsm/L or less means that the osmotic pressure of the specimen in which the blood collected in the blood collection container and the above aqueous solution are mixed is not excessively small and not excessively large. When the osmotic pressure of the above mixed liquid is the above lower limit or more and the above upper limit or less, cell death of CTCs can be suppressed, the cell morphology of CTCs can be maintained, and mixing of white blood cells can also be suppressed. Therefore, the recovery rate of CTCs can be increased even when a specimen stored for several days after blood collection is used.

The osmotic pressure of the above mixed liquid in which the above physiological saline and the above aqueous solution are mixed is preferably 275 mOsm/L or more, more preferably 280 mOsm/L or more, still more preferably 285 mOsm/L or more, particularly preferably 290 mOsm/L or more, preferably 340 mOsm/L or less, more preferably 330 mOsm/L or less, still more preferably 320 mOsm/L or less, yet more preferably 310 mOsm/L or less, particularly preferably 300 mOsm/L or less. When the osmotic pressure of the above mixed liquid is the above lower limit or more and the above upper limit or less, cell death of CTCs can be further suppressed, and the cell morphology of CTCs can be further favorably maintained. Therefore, the recovery rate of CTCs can be further increased even when a specimen stored for several days after blood collection is used.

The osmotic pressure of the above mixed liquid is measured by a freezing point depression method using an osmotic pressure meter (for example, "OM-6060" manufactured by ARKRAY, Inc.).

In the above blood collection container, 3 mL or more, more preferably 4 mL or more, preferably 11 mL or less, more preferably 10 mL or less of blood is collected with respect to 1 mL of the above aqueous solution accommodated. In this case, the effect of the present invention can be more effectively exhibited without excessively diluting the blood.

The shape of the above blood collection container main body is not particularly limited. The above blood collection container main body is preferably a bottomed tubular container.

The material of the above blood collection container main body is not particularly limited. Examples of the material of the above blood collection container main body include thermoplastic resins such as polyethylene, polypropylene, polystyrene, polyethylene terephthalate, polymethyl methacrylate, and polyacrylonitrile; thermosetting resins such as unsaturated polyester resin, epoxy resin, and epoxy-acrylate resin; modified natural resins such as cellulose acetate, cellulose propionate, ethyl cellulose, and ethyl chitin; and glasses such as silicate glasses such as soda lime glass, phosphosilicate glass, and borosilicate glass, and quartz glass. Only one kind of the material of the above blood collection container main body may be used, or two or more kinds thereof may be used in combination.

From the viewpoint of improving container strength, oxygen barrier property, and visibility of the contents, the material of the above blood collection container main body is preferably polyethylene terephthalate.

The above blood collection container preferably includes a plug. The above plug is preferably attached to the opening of the blood collection container main body. As the above plug, a conventionally known plug can be used. The above plug is preferably formed of a material and a shape that can be air-tightly and liquid-tightly attached to the opening of the blood collection container main body. The above plug is preferably configured such that a blood sampling needle can be inserted therethrough.

Examples of the above plug include a plug having a shape fitted to the opening of the blood collection container main body and a sheet-like seal plug.

The above plug may be a plug including a plug main body such as a rubber plug and a cap member made of plastic or the like. In this case, it is possible to suppress the risk that blood comes into contact with a human body when the plug is pulled out from the opening of the blood collection container main body after blood collection.

Examples of the material of the above plug (or the above plug main body) include synthetic resin, elastomer, rubber, and metal foil. Examples of the above rubber include butyl rubber and halogenated butyl rubber. Examples of the above metal foil include an aluminum foil. From the viewpoint of enhancing sealing property, the material of the above plug is preferably butyl rubber. The above plug (or the above plug main body) is preferably a butyl rubber plug.

The internal pressure of the above blood collection container is not particularly limited. The above blood collection container can also be used as a vacuum blood collection tube sealed by the above plug after the inside is evacuated. In the case of the vacuum blood collection tube, it is possible to easily collect a certain amount of blood regardless of a technical difference of a blood collector.

<Cell isolation container>

The above cell isolation container includes a cell isolation container main body and the above cell isolation material accommodated in the above cell isolation container main body. Therefore, the above cell isolation material is accommodated in the above cell isolation container.

The accommodated location of the above cell isolation material is not particularly limited as long as the accommodated location is in the above cell isolation container main body. The above cell isolation material may be disposed at the bottom of the above cell isolation container main body, or may be disposed on an inner wall surface of the above cell isolation container main body. From the viewpoint of more effectively exhibiting the effect of the present invention, it is preferable that the above cell isolation material is accommodated in the bottom of the above cell isolation container main body.

The shape of the above cell isolation container main body is not particularly limited. The above cell isolation container main body is preferably a bottomed tubular container.

The material of the above cell isolation container main body is not particularly limited. As the material of the above cell isolation container main body, those described in the section of the material of the above blood collection container main body can be used.

From the viewpoint of effectively suppressing cell adsorption and further improving the recovery rate of CTCs, the material of the above cell isolation container main body is preferably polypropylene.

The above cell isolation container preferably includes a plug. The above plug is preferably attached to the opening of the cell isolation container main body. As the above plug, a conventionally known plug can be used. The above plug is preferably formed of a material and a shape that can be air-tightly and liquid-tightly attached to the opening of the cell isolation container main body. The above plug may be configured such that a needle can be inserted therethrough.

As the above plug, those described in the section of the plug of the above blood collection container can be used.

<Hemagglutinating agent>

The above CTC isolation kit preferably includes a hemagglutinating agent. The above hemagglutinating agent is preferably accommodated in a container. That is, the above CTC isolation kit preferably includes a third container accommodating a hemagglutinating agent. By using a hemagglutinating agent, when the cell isolation container is centrifuged, unnecessary blood cells such as red blood cells and white blood cells easily move downward from the cell isolation material, and CTCs and blood cells can be more favorably isolated.

The above hemagglutinating agent is not particularly limited as long as it is an agent capable of enhancing the purity of CTCs by selectively agglutinating blood cells other than CTCs and easily precipitating the blood cells by centrifugation operation. Only one kind of the above hemagglutinating agent may be used, or two or more kinds thereof may be used in combination.

The above hemagglutinating agent may contain an antibody having an antigen recognition site that binds to an antigen specific to a white blood cell surface, or may include an antibody having an antigen recognition site that binds to an antigen specific to a red blood cell surface. The above hemagglutinating agent may contain an antibody complex having both an antigen recognition site that binds to an antigen specific to a white blood cell surface and an antigen recognition site that binds to an antigen specific to a red blood cell surface. The above hemagglutinating agent may be a mixture thereof.

The above hemagglutinating agent is preferably an antibody complex having both an antigen recognition site that binds to an antigen specific to a white blood cell surface and an antigen recognition site that binds to an antigen specific to a red blood cell surface. The above hemagglutinating agent is preferably an antibody complex of an anti-white blood cell antibody and an anti-red blood cell antibody.

Examples of commercially available products of the above hemagglutinating agent include "RosetteSep human CD45 depletion cocktail" manufactured by STEMCELL Technologies Inc.

In addition, examples of another hemagglutinating agent having an agglutination action include a hemagglutinating agent in which an anti-white blood cell antibody or an anti-red blood cell antibody is physically bound or chemically bound to a carrier having a large specific gravity, and a hemagglutinating agent capable of physically adsorbing blood cells to an insoluble carrier such as microbeads.

(Circulating tumor cell (CTC) isolation method (1))

Hereinafter, a method for isolating CTCs in blood using the above-described CTC isolation kit will be described.

<Blood collection step>

The above CTC isolation method (1) includes a blood collection step of collecting blood in the above blood collection container accommodating the above aqueous solution to obtain a specimen in which the above blood and the above aqueous solution are mixed. In the above CTC isolation method (1), since the osmotic pressure of the above specimen can be appropriately reduced, the effect of the present invention is effectively exhibited. Examples of a method for mixing the above blood and the above aqueous solution include mixing by inversion.

After the above blood collection step, the specimen may be stored in the blood collection container, or the process may proceed to the next step without storing the specimen. The storage temperature is preferably 1°C or more and preferably 40°C or less.

<Addition step>

The above CTC isolation method (1) includes an addition step of adding the above specimen to the above cell isolation container accommodating the above cell isolation material.

In the above addition step, the entire amount of the specimen in the above blood collection container may be added to the above cell isolation container, or a part of the specimen in the above blood collection container may be added to the above cell isolation container. The amount of the specimen added to the cell isolation container in the above addition step can be, for example, 1 mL or more and 11 mL or less.

In the above addition step, it is preferable to add the above specimen collected from the above blood collection container to the above cell isolation container through a filter. Addition through a filter can effectively remove aggregates present in the specimen.

The pore size of the above filter is preferably 20 μm or more, more preferably 40 μm or more, preferably 100 μm or less, more preferably 70 μm or less. When the above pore size is the above lower limit or more and the above upper limit or less, aggregates present in the specimen can be effectively removed, and CTCs can be favorably added to the cell isolation container.

Examples of commercially available products of the above filter include "pluriSelect" manufactured by pluriSelect Life Science UG & Co, KG.

After the above addition step, the specimen may be stored in the cell isolation container, or the process may proceed to the next step without storing the specimen. The storage temperature is preferably 1°C or more and preferably 40°C or less.

<Centrifugation step>

The above CTC isolation method (1) includes a centrifugation step of centrifuging the above cell isolation container to which the above specimen has been added. By the centrifugation step, blood cell components move to a lower side of a partition formed by the cell isolation material, and plasma and CTCs move to an upper side.

In the above centrifugation step, it is preferable to centrifuge a cell isolation container accommodating a liquid in which a hemagglutinating agent and the above specimen are mixed. In the above centrifugation step, it is preferable that the above hemagglutinating agent is added to the above cell isolation container accommodating the above specimen, and then the cell isolation container is centrifuged. By using a hemagglutinating agent, unnecessary blood cells such as red blood cells and white blood cells easily move downward from the cell isolation material, and CTCs and blood cells can be more favorably isolated.

Examples of the condition of the above centrifugation in the above centrifugation step include a condition of performing centrifugation at 400 G or more and 4000 G or less for 10 minutes or more and 120 minutes or less.

<Recovery step>

The above CTC isolation method (1) includes a recovery step of recovering circulating tumor cells (CTCs) isolated in plasma. After the centrifugation step, CTCs are deposited on a partition formed by the cell isolation material or are present in plasma. Therefore, it is preferable that plasma is gently stirred by pipetting to suspend CTCs deposited on the partition, and then the CTCs are recovered.

(Circulating tumor cell (CTC) isolation container)

The above CTC isolation container includes a container main body, the above aqueous solution accommodated in the above container main body, and the above cell isolation material accommodated in the above container main body. Therefore, the above aqueous solution and the above cell isolation material are accommodated in the above CTC isolation container. In addition, a predetermined amount of blood is collected in the above CTC isolation container.

The amount of the aqueous solution accommodated in the above container main body is appropriately changed according to the size of the container main body, the amount of blood collected, and the like. The amount of the aqueous solution accommodated in the above container main body is preferably 0.1 mL or more, more preferably 0.5 mL or more, still more preferably 0.7 mL or more, preferably 5 mL or less, more preferably 3 mL or less, still more preferably 2.5 mL or less. When the above amount of the aqueous solution is the above lower limit or more and the above upper limit or less, the effect of the present invention can be more effectively exhibited without excessively diluting the blood.

The above CTC isolation container is a CTC isolation container in which a predetermined amount of blood is collected. The above predetermined amount of the above blood is appropriately changed depending on the size, internal pressure, and the like of the CTC isolation container. The above predetermined amount of the above blood may be 1 mL or more, 2 mL or more, 4 mL or more, 12 mL or less, 11 mL or less, or 10 mL or less.

Physiological saline in an amount equivalent to the predetermined amount of the blood collected in the above CTC isolation container is collected in the above CTC isolation container to obtain a mixed liquid in which the above physiological saline and the above aqueous solution are mixed. For example, in the CTC isolation container in which 5 mL of blood is collected, 5 mL of physiological saline is collected in the CTC isolation container and mixed by inversion or the like to mix the above physiological saline and the above aqueous solution to obtain a mixed liquid. From the viewpoint of exhibiting the effect of the present invention, in the above CTC isolation container, the osmotic pressure of the above mixed liquid in which the above physiological saline and the above aqueous solution are mixed is 270 mOsm/L or more and 350 mOsm/L or less. The fact that the osmotic pressure of the above mixed liquid is 270 mOsm/L or more and 350 mOsm/L or less means that the osmotic pressure of the specimen in which the blood collected in the CTC isolation container and the above aqueous solution are mixed is not excessively small and not excessively large. When the osmotic pressure of the above mixed liquid is the above lower limit or more and the above upper limit or less, cell death of CTCs can be suppressed, the cell morphology of CTCs can be maintained, and mixing of white blood cells can also be suppressed. Therefore, the recovery rate of CTCs can be increased even when a specimen stored for several days after blood collection is used.

The osmotic pressure of the above mixed liquid in which the above physiological saline and the above aqueous solution are mixed is preferably 275 mOsm/L or more, more preferably 280 mOsm/L or more, still more preferably 285 mOsm/L or more, particularly preferably 290 mOsm/L or more, preferably 340 mOsm/L or less, more preferably 330 mOsm/L or less, still more preferably 320 mOsm/L or less, yet more preferably 310 mOsm/L or less, particularly preferably 300 mOsm/L or less. When the osmotic pressure of the above mixed liquid is the above lower limit or more and the above upper limit or less, cell death of CTCs can be further suppressed, and the cell morphology of CTCs can be further favorably maintained. Therefore, the recovery rate of CTCs can be further increased even when a specimen stored for several days after blood collection is used.

The osmotic pressure of the above mixed liquid is measured by a freezing point depression method using an osmotic pressure meter (for example, "OM-6060" manufactured by ARKRAY, Inc.).

In the above CTC isolation container, 3 mL or more, more preferably 4 mL or more, preferably 11 mL or less, more preferably 10 mL or less of blood is collected with respect to 1 mL of the above aqueous solution accommodated. In this case, the effect of the present invention can be more effectively exhibited without excessively diluting the blood.

The accommodated location of the above cell isolation material is not particularly limited as long as the accommodated location is in the above container main body. The above cell isolation material may be disposed at the bottom of the above container main body, or may be disposed on an inner wall surface of the above container main body. From the viewpoint of more effectively exhibiting the effect of the present invention, it is preferable that the above cell isolation material is accommodated in the bottom of the above container main body.

The shape of the above container main body is not particularly limited. The above container main body is preferably a bottomed tubular container.

The material of the above container main body is not particularly limited. As the material of the above container main body, those described in the section of the material of the above blood collection container main body can be used.

The above CTC isolation container preferably includes a plug. The above plug is preferably attached to the opening of the CTC isolation container main body. As the above plug, a conventionally known plug can be used. The above plug is preferably formed of a material and a shape that can be air-tightly and liquid-tightly attached to the opening of the CTC isolation container main body. The above plug may be configured such that a needle can be inserted therethrough.

As the above plug, those described in the section of the plug of the above blood collection container can be used.

The internal pressure of the above CTC isolation container is not particularly limited. The above CTC isolation container can also be used as a vacuum blood collection tube sealed by the above plug after the inside is evacuated. In the case of the vacuum blood collection tube, it is possible to easily collect a certain amount of blood regardless of a technical difference of a blood collector.

The above CTC isolation container can also be used together with the above-described hemagglutinating agent. That is, provided herein is a circulating tumor cell isolation kit (CTC isolation kit) including the above CTC isolation container and the above hemagglutinating agent. The CTC isolation kit including the above CTC isolation container preferably includes a second container accommodating a hemagglutinating agent. By using a hemagglutinating agent, when the CTC isolation container is centrifuged, unnecessary blood cells such as red blood cells and white blood cells easily move downward from the cell isolation material, and CTCs and blood cells can be more favorably isolated.

(Circulating tumor cell (CTC) isolation method (2))

Hereinafter, a method for isolating CTCs in blood using the above-described CTC isolation container will be described.

<Blood collection step>

The above CTC isolation method (2) includes a blood collection step of collecting blood in the above CTC isolation container to obtain a specimen in which the above blood and the above aqueous solution are mixed. In the above CTC isolation method (2), since the osmotic pressure of the above specimen can be reduced, the effect of the present invention is effectively exhibited. Examples of a method for mixing the above blood and the above aqueous solution include mixing by inversion.

After the above blood collection step, the specimen may be stored in the CTC isolation container, or the process may proceed to the next step without storing the specimen. The storage temperature is preferably 1°C or more and preferably 40°C or less.

<Centrifugation step>

The above CTC isolation method (2) includes a centrifugation step of centrifuging the above CTC isolation container accommodating the above specimen. By the centrifugation step, blood cell components move to a lower side of a partition formed by the cell isolation material, and plasma and CTCs move to an upper side.

In the above centrifugation step, it is preferable to centrifuge a CTC isolation container accommodating a liquid in which a hemagglutinating agent and the above specimen are mixed. In the above centrifugation step, it is preferable that the above hemagglutinating agent is added to the above CTC isolation container accommodating the above specimen, and then the CTC isolation container is centrifuged. By using a hemagglutinating agent, unnecessary blood cells such as red blood cells and white blood cells easily move downward from the cell isolation material, and CTCs and blood cells can be more favorably isolated.

Examples of the condition of the above centrifugation in the above centrifugation step include a condition of performing centrifugation at 400 G or more and 4000 G or less for 10 minutes or more and 120 minutes or less.

<Recovery step>

The above CTC isolation method (2) includes a recovery step of recovering circulating tumor cells (CTCs) isolated in plasma. After the centrifugation step, CTCs are deposited on a partition formed by the cell isolation material or are present in plasma. Therefore, it is preferable that plasma is gently stirred by pipetting to suspend CTCs deposited on the partition, and then the CTCs are recovered.

(Circulating tumor cell (CTC) detection method)

Examples of a method for detecting CTCs isolated by the above CTC isolation method (1) or (2) include a method in which CTCs are fluorescently labeled, and then the fluorescently labeled CTCs are detected using a detection instrument.

That is, the above CTC detection method preferably includes a step of isolating CTCs from blood, a step of fluorescently labeling the isolated CTCs, and a step of detecting the fluorescently labeled CTCs. In the above step of isolating CTCs from blood, it is preferable to use the above-described CTC isolation method (1) or (2).

The method for fluorescently labeling CTCs is not particularly limited, and a conventionally known method can be used. For example, CTCs can be fluorescently labeled by bringing a labeled antibody that specifically binds to an antigen possessed by a CTC into contact with the CTC. In addition, it is also possible to fluorescently label CTCs using a reagent containing a virus that infects CTCs, reacts with telomerase activity specific to CTCs to proliferate, and fluoresces.

The fluorescently labeled CTCs can be detected using a detection instrument such as a fluorescence microscope or a flow cytometer.

Hereinafter, the present invention will be described in more detail with reference to Examples. The present invention is not limited only to the following Examples.

The following were prepared as solutes of an aqueous solution.

(Anticoagulant)
Dipotassium ethylenediaminetetraacetate dihydrate (EDTA2K·2H$_2$O)
(Compound not corresponding to low molecular weight compound (A))
Sodium chloride
(Low molecular weight compound (A))

Ammonium sulfate
Trehalose 2H$_2$O

(High molecular weight compound (B))

Dextran (number average molecular weight: 40,000)
Polyethylene glycol (number average molecular weight: 4,000)

As materials of a composition for cell isolation, the following were prepared.

(Material of organic component having fluidity at 25°C)

(Meth)acrylic resin:

2-Ethylhexyl acrylate and butyl acrylate were radically polymerized in the presence of an azo polymerization initiator by a solution polymerization method to obtain a (meth)acrylic acid ester polymer having fluidity at 25°C.

(Inorganic fine powder)

Hydrophilic silica (fine powder silica, "200CF" manufactured by NIPPON AEROSIL CO., LTD.)
Hydrophobic silica (fine powder silica, "RX200" manufactured by NIPPON AEROSIL CO., LTD.)
Calcium carbonate powder ("Socal UP-G" manufactured by Imerys S.A.)

(Other components)
Silicone oil ("SF8410" manufactured by Dow Corning Toray Co., Ltd.)

Production of composition for cell isolation A:

A (meth)acrylic resin (91.9 parts by weight), 1.00 part by weight of hydrophilic silica, 5.0 parts by weight of hydrophobic silica, 1.95 parts by weight of calcium carbonate powder, and 0.15 parts by weight of silicone oil were mixed to produce composition for cell isolation A.

Production of composition for cell isolation B:

A (meth)acrylic resin (92.4 parts by weight), 0.55 parts by weight of hydrophilic silica, 6.9 parts by weight of hydrophobic silica, and 0.15 parts by weight of silicone oil were mixed to produce composition for cell isolation B.

One drop of the obtained composition for cell isolation was sequentially added into saline at 25°C whose specific gravity was adjusted stepwise at intervals of 0.002, and the specific gravity was measured by flotation and sedimentation in the saline. The specific gravity at 25°C of the obtained composition for cell isolation is shown in the following table.

As a blood collection container main body in a CTC isolation kit and a container main body in a CTC isolation container, the following was prepared.

PET bottomed tube (polyethylene terephthalate tube) having a length of 100 mm and an inner diameter of the opening of 14 mm

As a cell isolation container main body in the CTC isolation kit, the following was prepared.

PP bottomed tube (polypropylene tube) having a length of 119 mm and an inner diameter of the opening of 14 mm

(Example 1)

(1) Production of blood collection container

The components shown in Table 1 were dissolved in water (water for injection) to obtain an aqueous solution. The types and concentrations of the components of the obtained aqueous solution are shown in Table 1.

The obtained aqueous solution (1 mL) was added into a blood collection container main body. The inside of the blood collection container was decompressed so that the blood collection amount was 5 mL, and sealed with a butyl rubber plug. In this way, a blood collection container was produced.

(2) Production of cell isolation container

Composition for cell isolation A (1.8 g) was accommodated in the bottom of a cell isolation container main body, and sealed with a screw cap. In this way, a cell isolation container was produced.

In this way, a CTC isolation kit including a blood collection container and a cell isolation container was obtained.

(Examples 2 to 5 and Comparative Examples 1 and 2)

A CTC isolation kit including a blood collection container and a cell isolation container was obtained in the same manner as in Example 1 except that the composition of the aqueous solution and the type of the composition for cell isolation were changed as shown in Tables 1 and 2.

(Comparative Example 3)

(1) Production of blood collection container

An anticoagulant (33 parts by weight) was dissolved in 67 parts by weight of water to obtain a mixed liquid. In addition, 23 mg of the obtained mixed liquid was applied to the inner wall surface of a blood collection container main body and dried. The inside of the blood collection container was decompressed so that the blood collection amount was 5 mL, and sealed with a butyl rubber plug. In this way, a blood collection container was produced.

(2) Production of cell isolation container

A cell isolation container was produced in the same manner as in Example 1.

In this way, a CTC isolation kit including a blood collection container and a cell isolation container was obtained.

(Example 6)

Production of CTC isolation container:

The components shown in Table 3 were dissolved in water (water for injection) to obtain an aqueous solution. The types and concentrations of the components of the obtained aqueous solution are shown in Table 3.

Composition for cell isolation A (1.8 g) was accommodated in the bottom of a container main body. In addition, 1 mL of the obtained aqueous solution was added into the container main body. In addition, the inside of the container was decompressed so that the blood collection amount was 5 mL, and sealed with a butyl rubber plug. In this way, a CTC isolation container was produced.

(Examples 7 to 10 and Comparative Examples 4 and 5)

A CTC isolation container was produced in the same manner as in Example 6 except that the composition of the aqueous solution and the type of the composition for cell isolation were changed as shown in Tables 3 and 4.

(Comparative Example 6)

Composition for cell isolation A (1.8 g) was accommodated in the bottom of a container main body. An anticoagulant (33 parts by weight) was dissolved in 67 parts by weight of water to obtain a mixed liquid. In addition, 23 mg of the obtained mixed liquid was applied to the inner wall surface of the container main body and dried. The inside of the container was decompressed so that the blood collection amount was 5 mL, and sealed with a butyl rubber plug. In this way, a CTC isolation container was produced.

(Evaluation)

(1) Osmotic pressure of mixed liquid

(1-1) Test procedure when CTC isolation kit was used

In the obtained blood collection container, 5 mL of physiological saline was collected. After the physiological saline was collected, the physiological saline was mixed by inversion, and the physiological saline and the aqueous solution accommodated in the blood collection container were mixed to obtain a mixed liquid. The osmotic pressure of the obtained mixed liquid was measured by a freezing point depression method using an osmotic pressure meter ("OM-6060" manufactured by ARKRAY, Inc.). In the same manner, the osmotic pressure of the aqueous solution accommodated in the blood collection container was also measured.

(1-2) Test procedure when CTC isolation container was used

In the obtained CTC isolation container, 5 mL of physiological saline was collected. After the physiological saline was collected, the physiological saline was mixed by inversion, and the physiological saline and the aqueous solution accommodated in the CTC isolation container were mixed to obtain a mixed liquid. The osmotic pressure of the obtained mixed liquid was measured by a freezing point depression method using an osmotic pressure meter ("OM-6060" manufactured by ARKRAY, Inc.). In the same manner, the osmotic pressure of the aqueous solution accommodated in the CTC isolation container was also measured.

(2) Recovery rate of CTCs

(2-1) Test procedure when CTC isolation kit was used (blood collection step to centrifugation step)

Using the obtained CTC isolation kits, a test for determining the recovery rate of CTCs was performed by the following procedure. In each of Examples and Comparative Examples, two sets of CTC isolation kits were used. In the first set of CTC isolation kits, the blood collection step to the CTC detection step were performed on the same day. On the other hand, in the second set of CTC isolation kits, the blood collection step to the addition step were performed on the same day, then the specimen was stored for 4 days, and then the centrifugation step to the CTC detection step were performed on the same day.

<Blood collection step>

In a blood collection container, 5 mL of blood was collected. After the blood was collected, the blood was mixed by inversion to obtain a specimen in which the blood and the aqueous solution accommodated in the blood collection container were mixed.

<Addition step>

To a cell isolation container, 1 mL of the obtained specimen was added.
Next, to the cell isolation container, Cell Line (MCF-7), which is a CTC, was added in an amount of 50 cells/1 mL of blood. As described above, in the first set of CTC isolation kits, the process directly proceeded to the next step. On the other hand, in the second set of CTC isolation kits, after the addition of CTCs, the cell isolation container was stored at 4°C for 4 days, and the process proceeded to the next step.

<Centrifugation step>

As a hemagglutinating agent, "RosetteSep human CD45 depletion cocktail" manufactured by STEMCELL Technologies Inc. was prepared. In Examples 1 to 3 and 5 and Comparative Examples 1 to 3, 50 μL of the hemagglutinating agent was added to the cell isolation container to which CTCs had been added, and the mixture was allowed to stand for 20 minutes. Then, the cell isolation container was centrifuged at 25°C and 1500 G for 20 minutes. In Example 4, the cell isolation container was centrifuged at 25°C and 1500 G for 20 minutes without using a hemagglutinating agent. After centrifugation, plasma was located above the partition formed by the composition for cell isolation, and CTCs were deposited on the partition.

(2-2) Test procedure when CTC isolation container was used (blood collection step to centrifugation step)

Using the obtained CTC isolation containers, a test for determining the recovery rate of CTCs was performed by the following procedure. In each of Examples and Comparative Examples, two CTC isolation containers were used. In the first CTC isolation container, the blood collection step to the CTC detection step were performed on the same day. On the other hand, in the second CTC isolation container, after the blood collection step, the specimen was stored for 4 days, and then the centrifugation step to the CTC detection step were performed on the same day.

<Blood collection step>

In a CTC isolation container, 5 mL of blood was collected. After the blood was collected, the blood was mixed by inversion to obtain a specimen in which the blood and the aqueous solution accommodated in the CTC isolation container were mixed.
Next, to the CTC isolation container, Cell Line (MCF-7), which is a CTC, was added in an amount of 50 cells/1 mL of blood. As described above, in the first CTC isolation container, the process directly proceeded to the next step. On the other hand, in the second CTC isolation container, after the addition of CTCs, the CTC isolation container was stored at 4°C for 4 days, and the process proceeded to the next step.

<Centrifugation step>

As a hemagglutinating agent, "RosetteSep human CD45 depletion cocktail" manufactured by STEMCELL Technologies Inc. was prepared. In Examples 6 to 8 and 10 and Comparative Examples 4 to 6, 250 μL of the hemagglutinating agent was added to the CTC isolation container to which CTCs had been added, and the mixture was allowed to stand for 20 minutes. Then, the CTC isolation container was centrifuged at 25°C and 1500 G for 20 minutes. In Example 9, the CTC isolation container was centrifuged at 25°C and 1500 G for 20 minutes without using a hemagglutinating agent. After centrifugation, plasma was located above the partition formed by the composition for cell isolation, and CTCs were deposited on the partition.

(2-3) Test procedure when CTC isolation kit and CTC isolation container were used (common)

<Recovery step>

Plasma was gently stirred by pipetting to suspend the CTCs deposited on the partition in the plasma, and then the plasma was recovered in an Eppendorf tube. Next, 1 mL of a hemolytic agent per 1 mL of the plasma was added to the recovered plasma. The mixed liquid of the plasma and the hemolytic agent was stirred at 25°C for 15 minutes, and then centrifuged at 25°C and 500 G for 5 minutes to hemolyze red blood cells mixed in the plasma. The supernatant was removed, the pellet was suspended in a mixed liquid of PBS and a 10% BSA solution, and hemolysis treatment was performed again. After the hemolysis treatment, the supernatant was removed, and the pellet was suspended in 80 μL of a mixed liquid of PBS and a 10% BSA solution to obtain a suspension.

<Staining of CTCs and fluorescence microscopic observation>

To 80 μL of the suspension was added 20 μL of FcR Blocking Reagent (manufactured by Miltenyi Biotec), and the mixture was stirred with a vortex mixer and then allowed to stand in a dark room at 4°C for 10 minutes. Then, 2 μL of CD326 (EpCAM)-FITC antibody (human) (manufactured by Miltenyi Biotec) was added to the suspension, and the mixture was pipetted lightly and allowed to stand in a dark room at 4°C for 10 minutes. Then, the suspension was washed with 1 mL of a mixed liquid of PBS and a 10% BSA solution. This liquid was added to a 96-well plate, and cells were seeded on the plate. Then, the number of stained cells was counted by performing fluorescence microscopic observation.

The recovery rate of CTCs was calculated by the following formula. In addition, the difference between the recovery rate of CTCs when the blood collection step to the CTC detection step were performed on the same day (storage day 0) and the recovery rate of CTCs when the specimen was stored for four days was defined as the amount of decrease in the recovery rate of CTCs.

$$\text{Recovery rate of CTCs (\%) = A/B} \times 100$$

A: Number of Cell Lines (MCF-7) observed with fluorescence microscope
B: Number of Cell Lines (MCF-7) added to blood

(3) Fluorescence intensity of recovered CTCs

The fluorescence intensity (0) of CTCs recovered when the blood collection step to the CTC detection step were performed on the same day (storage day 0) and the fluorescence intensity (4) of CTCs recovered when the specimen was stored for 4 days were confirmed using fluorescence microscopic observation. Specifically, the recovered cells were irradiated with light (LED light source) having a wavelength of 470 nm, and the fluorescence intensity of CTCs was observed. A cell having a strong fluorescence intensity is a living cell, and a cell having a weak fluorescence intensity is a dead cell or a cell in which a surface antigen has disappeared.

<Criteria for determination of fluorescence intensity of recovered CTCs>

∘: Fluorescence intensity (4) maintains intensity equivalent to fluorescence intensity (0)
×: Fluorescence intensity (4) is clearly less than fluorescence intensity (0)

(4) Rate of change in number of white blood cells remaining on partition

(4-1) Test procedure when cell isolation container in CTC isolation kit was used (blood collection step to centrifugation step)

Using the obtained CTC isolation kit, a test for determining the rate of change in the number of white blood cells remaining on the partition formed by the composition for cell isolation was performed by the following procedure. Two sets of CTC isolation kits were used. In the first set of CTC isolation kits, the blood collection step to measurement of the number of white blood cells were performed on the same day. On the other hand, in the second set of CTC isolation kits, the blood collection step to the addition step were performed on the same day, then the specimen was stored for 4 days, and then the centrifugation step to measurement of the number of white blood cells were performed on the same day.

<Blood collection step>

In a blood collection container, 5 mL of blood was collected. After the blood was collected, the blood was mixed by inversion to obtain a specimen in which the blood and the aqueous solution accommodated in the blood collection container were mixed.

<Addition step>

To a cell isolation container, 1 mL of the obtained specimen was added.

<Centrifugation step>

As a hemagglutinating agent, "RosetteSep human CD45 depletion cocktail" manufactured by STEMCELL Technologies Inc. was prepared. The hemagglutinating agent (50 uL) was added to the cell isolation container, and the mixture was allowed to stand for 20 minutes. Then, the cell isolation container was centrifuged at 25°C and 1500 G for 20 minutes. After centrifugation, plasma was located above the partition formed by the composition for cell isolation, and white blood cells were deposited on the partition.

(4-2) Test procedure when CTC isolation container was used (blood collection step to centrifugation step)

Using the obtained CTC isolation container, a test for determining the rate of change in the number of white blood cells remaining on the partition formed by the composition for cell isolation was performed by the following procedure. Two CTC isolation containers were used. In the first CTC isolation container, the blood collection step to measurement of the number of white blood cells were performed on the same day. On the other hand, in the second CTC isolation container, after the blood collection step, the specimen was stored for 4 days, and then the centrifugation step to measurement of the number of white blood cells were performed on the same day.

<Blood collection step>

In a CTC isolation container, 5 mL of blood was collected. After the blood was collected, the blood was mixed by inversion to obtain a specimen in which the blood and the aqueous solution accommodated in the CTC isolation container were mixed.

<Centrifugation step>

As a hemagglutinating agent, "RosetteSep human CD45 depletion cocktail" manufactured by STEMCELL Technologies Inc. was prepared. The hemagglutinating agent (250 uL) was added to the CTC isolation container, and the mixture was allowed to stand for 20 minutes. Then, the CTC isolation container was centrifuged at 25°C and 1500 G for 20 minutes. After centrifugation, plasma was located above the partition formed by the composition for cell isolation, and white blood cells were deposited on the partition.

(4-3) Test procedure when CTC isolation kit and CTC isolation container were used (common)

<Recovery step>

Plasma was gently stirred by pipetting to suspend the white blood cells deposited on the partition in the plasma, and then the plasma was recovered in an Eppendorf tube.

<Measurement of number of white blood cells>

The number of white blood cells in the recovered liquid was measured using a multi-item automatic blood cell analyzer ("XN series" manufactured by SYSMEX CORPORATION).

The rate of change in the number of white blood cells remaining on the partition was calculated by the following formula from the number of white blood cells (0) on the partition when the blood collection step to measurement of the number of white blood cells was performed on the same day (storage day 0) and the number of white blood cells (4) on the partition when the specimen was stored for 4 days. In the tables, "N/A" means that the number of white blood cells on the partition was small and the number of white blood cells was below the detection limit in the measurement with the multi-item automatic blood cell analyzer.

Rate of change in number of white blood cells remaining on partition (%) = (A - B)/B $\times$ 100

A: Number of white blood cells (4) on partition
B: Number of white blood cells (0) on partition

The configurations and results are shown in Tables 1 to 4 below. In the tables, the concentration of the anticoagulant means not the concentration of EDTA2K·2H$_2$O but the concentration of EDTA2K. The content of the other components is a pure amount.

[Table 1]

| CTC isolation Kit | | | | | Example 1 | Example 2 | Example 3 | Example 4 | Example 5 |
|---|---|---|---|---|---|---|---|---|---|
| Blood collection container | Aqueous solution | | Sodium chloride | wt% | - | - | - | - | - |
| | | Low molecular weight compound (A) | Ammonium sulfate | wt% | 1.10 | - | 0.55 | 0.55 | - |
| | | | Trehalose (Compound having cell membrane protective action) | wt% | - | 8 | 3 | 3 | β |
| | | High molecular weight compound (B) | Dextran (Mn: 40,000) (Compound having cell membrane protective action) | wt% | - | 5 | 5 | 5 | - |
| | | | Polyethylene glycol (Mn: 4,000) (Compound having cell membrane protective action) | wt% | - | 7 | 7 | 7 | 7 |
| | | Anticoagulant | EDTA2K ·2H$_2$O | wt% | 0.7848 | 0.7848 | 0.7848 | 0.7848 | 0.7848 |
| | | Water for injection | | wt% | Remaining amount | Remaining amount | Remaining amount | Remaining amount | Remaining amount |
| | | Total | | wt% | 100 | 100 | 100 | 100 | 100 |
| | | Osmotic pressure of aqueous solution | | mOsm/L | 290 | 330 | 340 | 340 | 340 |
| | | Amount of aqueous solution accommodated in blood collection container | | mL | 1 | 1 | 1 | 1 | 1 |
| | Osmotic pressure of mixed liquid | | | mOsm/L | 286 | 293 | 294 | 294 | 294 |
| Cell isolation container | Composition for cell isolation | | Type | - | A | B | B | B | A |
| | | | Specific gravity at 25°C | - | 1.077 | 1.071 | 1.071 | 1.071 | 1.077 |
| Hemagglutinating agent | | | | | Used | Used | Used | Not used | Used |
| Evaluation | Recovery rate of CTCs | | Storage day 0 | % | 91 | 88 | 78 | 85 | 94 |
| | | | Storage day 4 | % | 65 | 71 | 72 | 78 | 72 |
| | | | Amount of decrease in recovery rate of CTCs | % | 26 | 17 | 6 | 7 | 22 |
| | Fluorescence intensity of recovered CTCs | | | - | ○ | ○ | ○ | ○ | ○ |
| | Rate of change in number of white blood cells remaining on partition | | | % | 40 | 30 | 0 | 25 | 290 |

[Table 2]

| CTC isolation Kit | | | | | Comparative Example 1 | Comparative Example 2 | Comparative Example 3 |
|---|---|---|---|---|---|---|---|
| Blood collection container | Aqueous solution | | Sodium chloride | wt% | - | 0.75 | Dry material layer of anticoagulant |
| | | Low molecular weight compound (A) | Ammonium sulfate | wt% | 10.00 | - | |
| | | | Trehalose (Compound having cell membrane protective action) | wt% | 3 | - | |
| | | High molecular weight compound (B) | Dextran (Mn: 40,000) (Compound having cell membrane protective action) | wt% | 5 | - | |
| | | | Polyethylene glycol (Mn: 4,000) (Compound having cell membrane protective action) | wt% | 7 | - | |
| | | Anticoagulant | EDTA2K·2H$_2$O | wt% | 0.7848 | 0.7848 | |
| | | Water for injection | | wt% | Remaining amount | Remaining amount | |
| | | Total | | wt% | 100 | 100 | |
| | | Osmotic pressure of aqueous solution | | mOsm/L | 2380 | 320 | |
| | | Amount of aqueous solution accommodated in blood collection container | | mL | 1 | 1 | 0 |
| | Osmotic pressure of mixed liquid | | | mOsm/L | 634 | 291 | - |
| Cell isolation container | Composition for cell isolation | | Type | - | A | A | A |
| | | | Specific gravity at 25°C | - | 1.077 | 1.077 | 1.077 |
| Hemagglutinating agent | | | | | Used | Used | Used |
| Evaluation | Recovery rate of CTCs | | Storage day 0 | % | 52 | 69 | 76 |
| | | | Storage day 4 | % | 2 | 45 | 49 |
| | | | Amount of decrease in recovery rate of CTCs | % | 50 | 24 | 27 |
| | Fluorescence intensity of recovered CTCs | | | - | ○ | × | × |
| | Rate of change in number of white blood cells remaining on partition | | | % | N/A | 350 | 1300 |

[Table 3]

| | | | Example 6 | Example 7 | Example 8 | Example 9 | Example 10 |
|---|---|---|---|---|---|---|---|
| CTC isolation container | Aqueous solution | Sodium chloride | wt% | - | - | - | - | - |
| | | | Low molecular weight compound (A) | Ammonium sulfate | wt% | 1.10 | - | 0.55 | 0.55 | - |
| | | | | Trehalose (Compound having cell membrane protective action) | wt% | - | 8 | 3 | 3 | β |
| | | High molecular weight compound (B) | Dextran (Mn: 40,000) (Compound having cell membrane protective action) | wt% | - | 5 | 5 | 5 | - |
| | | | Polyethylene glycol (Mn: 4,000) (Compound having cell membrane protective action) | wt% | - | 7 | 7 | 7 | 7 |
| | | Anticoagulant | EDTA2K · 2H$_2$O | wt% | 0.7848 | 0.7848 | 0.7848 | 0.7848 | 0.7848 |
| | | | Water for injection | wt% | Remaining amount | Remaining amount | Remaining amount | Remaining amount | Remaining amount |
| | | | Total | wt% | 100 | 100 | 100 | 100 | 100 |
| | | Osmotic pressure of aqueous solution | mOsm/L | 290 | 330 | 340 | 340 | 340 |
| | | Amount of aqueous solution accommodated in CTC isolation container | mL | 1 | 1 | 1 | 1 | 1 |
| | Composition for cell isolation | Type | - | A | B | B | B | A |
| | | Specific gravity at 25°C | - | 1.077 | 1.071 | 1.071 | 1.071 | 1.077 |
| | Osmotic pressure of mixed liquid | mOsm/L | 286 | 2 93 | 294 | 294 | 294 |
| Hemagglutinating agent | | | Used | Used | Used | Not used | Used |
| Evaluation | Recovery rate of CTCs | Storage day 0 | % | 80 | 79 | 90 | 93 | 78 |
| | | Storage day 4 | % | 55 | 60 | 75 | 72 | 61 |
| | | Amount of decrease in recovery rate of CTCs | % | 25 | 19 | 15 | 21 | 17 |
| | Fluorescence intensity of recovered CTCs | - | ○ | ○ | ○ | ○ | ○ |
| | Rate of change in number of white blood cells remaining on partition | % | 44 | -100 | 0 | 21 | 300 |

[Table 4]

| | | | | | Comparative Example 4 | Comparative Example 5 | Comparative Example 6 |
|---|---|---|---|---|---|---|---|
| CTC isolation container | Aqueous solution | | | Sodium chloride | wt% | - | 0.75 | Dry material layer of anticoagulant |
| | | Low molecular weight compound (A) | Ammonium sulfate | wt% | 10.00 | - | |
| | | | Trehalose (Compound having cell membrane protective action) | wt% | 3 | - | |
| | | High molecular weight compound (B) | Dextran (Mn: 40,000) (Compound having cell membrane protective action) | wt% | 5 | - | |
| | | | Polyethylene glycol (Mn: 4,000) (Compound having cell membrane protective action) | wt% | 7 | - | |
| | | Anticoagulant | EDTA2K·2H$_2$O | wt% | 0.7848 | 0.7848 | |
| | | Water for injection | | wt% | Remaining amount | Remaining amount | |
| | | Total | | wt% | 100 | 100 | |
| | | Osmotic pressure of aqueous solution | | mOsm/L | 2380 | 320 | |
| | | Amount of aqueous solution accommodated in CTC isolation container | | mL | 1 | 1 | 0 |
| | Composition for cell isolation | Type | | - | A | A | A |
| | | Specific gravity at 25°C | | - | 1.077 | 1.077 | 1.077 |
| | Osmotic pressure of mixed liquid | | | mOsm/L | 634 | 291 | - |
| Hemagglutinating agent | | | | | Used | Used | Used |
| Evaluation | Recovery rate of CTCs | Storage day 0 | | % | 67 | 65 | 77 |
| | | Storage day 4 | | % | 18 | 40 | 42 |
| | | Amount of decrease in recovery rate of CTCs | | % | 49 | 25 | 35 |
| | Fluorescence intensity of recovered CTCs | | | - | ○ | × | × |
| | Rate of change in number of white blood cells remaining on partition | | | % | N/A | 300 | 1200 |

EP 4 502 599 A1

27

**EXPLANATION OF SYMBOLS**

1: Blood collection container
2: Cell isolation container
5: CTC isolation kit
6: CTC isolation container
11: Blood collection container main body
12: Aqueous solution
13: Plug
21: Cell isolation container main body
22: Cell isolation material
23: Plug
61: Container main body
62: Aqueous solution
63: Cell isolation material
64: Plug

**Claims**

1. A circulating tumor cell isolation kit used for isolation of circulating tumor cells in blood, the circulating tumor cell isolation kit comprising:

a blood collection container accommodating an aqueous solution and collecting a predetermined amount of blood; and
a cell isolation container accommodating a cell isolation material having a specific gravity at 25°C of 1.065 or more and 1.080 or less,
the aqueous solution containing an anticoagulant and containing a low molecular weight compound having a molecular weight of 75 or more and 500 or less or a high molecular weight compound having a number average molecular weight of 2,000 or more and less than 200,000, and
when physiological saline in an amount equivalent to the predetermined amount of blood collected in the blood collection container is collected in the blood collection container to obtain a mixed liquid in which the physiological saline and the aqueous solution are mixed, an osmotic pressure of the mixed liquid being 270 mOsm/L or more and 350 mOsm/L or less.

2. The circulating tumor cell isolation kit according to claim 1, wherein the aqueous solution contains the low molecular weight compound.

3. The circulating tumor cell isolation kit according to claim 1, wherein the aqueous solution contains the high molecular weight compound.

4. The circulating tumor cell isolation kit according to claim 1, wherein the aqueous solution contains the low molecular weight compound and the high molecular weight compound.

5. The circulating tumor cell isolation kit according to any one of claims 1 to 4, wherein the low molecular weight compound contains trehalose or ammonium sulfate.

6. The circulating tumor cell isolation kit according to any one of claims 1 to 5, wherein the high molecular weight compound contains a compound having a cell membrane protective action.

7. The circulating tumor cell isolation kit according to any one of claims 1 to 6, wherein the high molecular weight compound is dextran, polyethylene glycol, or polyvinylpyrrolidone.

8. The circulating tumor cell isolation kit according to any one of claims 1 to 7, wherein the cell isolation material is a composition for cell isolation.

9. The circulating tumor cell isolation kit according to claim 8, wherein

the composition for cell isolation contains an organic component having fluidity at 25°C and an inorganic fine powder,

the organic component contains a resin, and

the inorganic fine powder contains fine powder silica.

10. The circulating tumor cell isolation kit according to any one of claims 1 to 9, further comprising a hemagglutinating agent.

11. A method for isolating circulating tumor cells in blood using the circulating tumor cell isolation kit according to any one of claims 1 to 10, the method comprising:

a blood collection step of collecting blood in the blood collection container accommodating the aqueous solution to obtain a specimen in which the blood and the aqueous solution are mixed;

an addition step of adding the specimen to the cell isolation container accommodating the cell isolation material;

a centrifugation step of centrifuging the cell isolation container to which the specimen has been added; and

a recovery step of recovering circulating tumor cells isolated in plasma.

12. The method for isolating circulating tumor cells according to claim 11, wherein in the centrifugation step, a cell isolation container accommodating a liquid in which a hemagglutinating agent and the specimen are mixed is centrifuged.

13. A circulating tumor cell isolation container used for isolation of circulating tumor cells in blood,

the circulating tumor cell isolation container being a circulating tumor cell isolation container in which a predetermined amount of blood is collected, the circulating tumor cell isolation container comprising:

a container main body;

an aqueous solution accommodated in the container main body; and

a cell isolation material accommodated in the container main body and having a specific gravity at 25°C of 1.065 or more and 1.080 or less,

the aqueous solution containing an anticoagulant and containing a low molecular weight compound having a molecular weight of 75 or more and 500 or less or a high molecular weight compound having a number average molecular weight of 2,000 or more and less than 200,000, and

when physiological saline in an amount equivalent to the predetermined amount of blood collected in the circulating tumor cell isolation container is collected in the circulating tumor cell isolation container to obtain a mixed liquid in which the physiological saline and the aqueous solution are mixed, an osmotic pressure of the mixed liquid being 270 mOsm/L or more and 350 mOsm/L or less.

14. The circulating tumor cell isolation container according to claim 13, wherein the aqueous solution contains the low molecular weight compound.

15. The circulating tumor cell isolation container according to claim 13, wherein the aqueous solution contains the high molecular weight compound.

16. The circulating tumor cell isolation container according to claim 13, wherein the aqueous solution contains the low molecular weight compound and the high molecular weight compound.

17. The circulating tumor cell isolation container according to any one of claims 13 to 16, wherein the low molecular weight compound contains trehalose or ammonium sulfate.

18. The circulating tumor cell isolation container according to any one of claims 13 to 17, wherein the high molecular weight compound contains a compound having a cell membrane protective action.

19. The circulating tumor cell isolation container according to any one of claims 13 to 18, wherein the high molecular weight compound is dextran, polyethylene glycol, or polyvinylpyrrolidone.

20. The circulating tumor cell isolation container according to any one of claims 13 to 19, wherein the cell isolation material is a composition for cell isolation.

21. The circulating tumor cell isolation container according to claim 20, wherein

the composition for cell isolation contains an organic component having fluidity at 25°C and an inorganic fine powder,

the organic component contains a resin, and

the inorganic fine powder contains fine powder silica.

22. A circulating tumor cell isolation kit, comprising:

the circulating tumor cell isolation container according to any one of claims 13 to 21; and

a hemagglutinating agent.

23. A method for isolating circulating tumor cells in blood using the circulating tumor cell isolation container according to any one of claims 13 to 21, the method comprising:

a blood collection step of collecting blood in the circulating tumor cell isolation container to obtain a specimen in which the blood and the aqueous solution are mixed;

a centrifugation step of centrifuging the circulating tumor cell isolation container accommodating the specimen; and

a recovery step of recovering circulating tumor cells isolated in plasma.

24. The method for isolating circulating tumor cells according to claim 23, wherein in the centrifugation step, a circulating tumor cell isolation container accommodating a liquid in which a hemagglutinating agent and the specimen are mixed is centrifuged.

[FIG. 1]

[FIG. 2]

6

64

61

62

63

**INTERNATIONAL SEARCH REPORT**

| International application No. |
| --- |
| **PCT/JP2022/038554** |

**A.    CLASSIFICATION OF SUBJECT MATTER**

*G01N 33/48*(2006.01)i; *C12M 1/00*(2006.01)i; *C12N 1/02*(2006.01)i; *C12N 5/09*(2010.01)i
FI:    G01N33/48 B; C12M1/00 A; C12N1/02; C12N5/09; G01N33/48 D

According to International Patent Classification (IPC) or to both national classification and IPC

**B.    FIELDS SEARCHED**

Minimum documentation searched (classification system followed by classification symbols)

G01N33/48-33/50; A61B5/15; C12M1/26

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| Y | JP 2021-500585 A (STRECK INC) 07 January 2021 (2021-01-07)<br>claims, paragraphs [0027]-[0028], [0049], [0062], [0067], [0069], [0076], [0081] | 1-4, 6-16, 18-24 |
| Y | JP 2019-211216 A (ARKRAY INC) 12 December 2019 (2019-12-12)<br>claims, paragraphs [0010], [0050], [0064]-[0067] | 1-2, 8-14, 20-24 |
| Y | JP 2005-501236 A (IMMUNIVEST CORPORATION) 13 January 2005 (2005-01-13)<br>claims, paragraphs [0006], [0016] | 1-4, 6-16, 18-24 |
| Y | WO 2015/046557 A1 (SEKISUI MEDICAL CO LTD) 02 April 2015 (2015-04-02)<br>claims, paragraphs [0037]-[0041] | 1-4, 6-16, 18-24 |
| A | WO 2021/182575 A1 (SEKISUI MEDICAL CO LTD) 16 September 2021 (2021-09-16)<br>entire text, all drawings | 1-24 |
| A | WO 2021/112119 A1 (SEKISUI MEDICAL CO LTD) 10 June 2021 (2021-06-10)<br>entire text, all drawings | 1-24 |
| A | JP 2020-523577 A (SUN, Chung-Chin) 06 August 2020 (2020-08-06)<br>entire text, all drawings | 1-24 |

☑ Further documents are listed in the continuation of Box C.        ☑ See patent family annex.

| | |
| --- | --- |
| *    Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
| --- | --- |
| **14 November 2022** | **29 November 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

# EP 4 502 599 A1

<table>
<tr><td colspan="3">INTERNATIONAL SEARCH REPORT</td><td colspan="2">International application No.<br>**PCT/JP2022/038554**</td></tr>
</table>

**C.    DOCUMENTS CONSIDERED TO BE RELEVANT**

| Category* | Citation of document, with indication, where appropriate, of the relevant passages | Relevant to claim No. |
|---|---|---|
| A | JP 2014-524027 A (BECTON DICKINSON AND COMPANY) 18 September 2014 (2014-09-18)<br>entire text, all drawings | 1-24 |
| A | JP 2015-506482 A (BECTON DICKINSON AND COMPANY) 02 March 2015 (2015-03-02)<br>entire text, all drawings | 1-24 |
| A | WO 2013/168767 A1 (KONICA MINOLTA, INC) 14 November 2013 (2013-11-14)<br>entire text, all drawings | 1-24 |
| A | JP 2007-271388 A (TSUCHIDA, Hidetoshi) 18 October 2007 (2007-10-18)<br>entire text, all drawings | 1-24 |
| A | JP 1-199158 A (SEITETSU KAGAKU CO LTD) 10 August 1989 (1989-08-10)<br>entire text, all drawings | 1-24 |

Form PCT/ISA/210 (second sheet) (January 2015)

EP 4 502 599 A1

## INTERNATIONAL SEARCH REPORT
### Information on patent family members

| | International application No. |
|---|---|
| | PCT/JP2022/038554 |

| Patent document cited in search report | | | Publication date (day/month/year) | Patent family member(s) | | | Publication date (day/month/year) |
|---|---|---|---|---|---|---|---|
| JP | 2021-500585 | A | 07 January 2021 | US 2021/0371848 A1 claims, paragraphs [0036]-[0037], [0058], [0071], [0076], [0078], [0085], [0090] WO 2019/079743 A1 EP 3697209 A1 CN 111372451 A CA 3079415 A1 | | | |
| JP | 2019-211216 | A | 12 December 2019 | US 2019/0365304 A1 claims, paragraphs [0014]-[0015], [0159], [0198]-[0222] EP 3575791 A1 CN 110547288 A | | | |
| JP | 2005-501236 | A | 13 January 2005 | WO 2003/018757 A2 claims, p. 2, lines 5-21, p. 5, lines 5-6 EP 1425294 A2 CA 2457891 A1 BR 212124 A CN 1571634 A KR 10-2004-0030984 A AU 2008288601 A1 | | | |
| WO | 2015/046557 | A1 | 02 April 2015 | US 2016/0223521 A1 claims, paragraphs [0045]-[0049] EP 3054001 A1 CN 105683353 A | | | |
| WO | 2021/182575 | A1 | 16 September 2021 | (Family: none) | | | |
| WO | 2021/112119 | A1 | 10 June 2021 | CN 114340493 A | | | |
| JP | 2020-523577 | A | 06 August 2020 | US 2021/0059240 A1 whole document WO 2018/227359 A1 | | | |
| JP | 2014-524027 | A | 18 September 2014 | US 2013/0183655 A1 whole document WO 2013/006550 A1 EP 2729068 A1 AU 2012279135 A1 CA 2841088 A1 CN 103764028 A KR 10-2014-0051281 A MX 2014000152 A NZ 619674 A RU 2014103327 A ZA 201400329 B SG 10201605495U A BR 112014000144 A2 | | | |
| JP | 2015-506482 | A | 02 March 2015 | US 2013/0209985 A1 whole document WO 2013/116702 A1 EP 2809289 A1 AU 2013214866 A1 CA 2862544 A1 | | | |

Form PCT/ISA/210 (patent family annex) (January 2015)

INTERNATIONAL SEARCH REPORT
Information on patent family members

International application No.

**PCT/JP2022/038554**

| Patent document cited in search report | | | Publication date (day/month/year) | Patent family member(s) | | | Publication date (day/month/year) |
|---|---|---|---|---|---|---|---|
| | | | | CN | 104244902 | A | |
| | | | | MX | 2014009256 | A | |
| | | | | IN | 6981DEN2014 | A | |
| WO | 2013/168767 | A1 | 14 November 2013 | US | 2015/0148212 | A1 | |
| | | | | whole document | | | |
| | | | | EP | 2848935 | A1 | |
| JP | 2007-271388 | A | 18 October 2007 | (Family: none) | | | |
| JP | 1-199158 | A | 10 August 1989 | (Family: none) | | | |

Form PCT/ISA/210 (patent family annex) (January 2015)

36

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021500585 T

- WO 2010132783 A1

**Non-patent literature cited in the description**

- **ZEINA HABLI** ; **WALID ALCHAMAA** ; **RAYA SAAB** ; **HUMAM KADARA** ; **MASSOUD L. KHRAICHE**. Circulating Tumor Cell Detection Technologies and Clinical Utility: Challenges and Opportunities. *Cancers*, 2020, vol. 12, 1930